# FASCICULE DE BREVET EUROPEEN

(11) **EP 2 945 706 B1**
(45) Date de publication et mention de la délivrance du brevet: **06.01.2021**
(21) Numéro de dépôt: 14705809.3
(22) Date de dépôt: 15.01.2014
(51) Int. Cl.: A61Q 5/02, A61Q 5/12, A61Q 19/10, A61K 8/02, A61K 8/46, A61K 8/73, A61K 8/81, A61K 8/898

(54) **COMPOSITION COSMÉTIQUE SOLIDE SOUPLE COMPRENANT DES TENSIOACTIFS ANIONIQUES ET DES AGENTS CONDITIONNEURS POLYMÉRIQUES, ET PROCÉDÉ DE TRAITEMENT COSMÉTIQUE**
FLEXIBLE, FESTE KOSMETISCHE ZUSAMMENSETZUNG MIT ANIONISCHEN TENSIDEN UND POLYMERKONDITIONIERUNGSMITTELN UND KOSMETISCHES BEHANDLUNGSVERFAHREN
SOFT SOLID COSMETIC COMPOSITION COMPRISING ANIONIC SURFACTANTS AND POLYMERIC CONDITIONING AGENTS, AND COSMETIC TREATMENT METHOD

(30) Priorité: 18.01.2013 FR 1350439; 27.02.2013 US 201361769810 P
(43) Date de publication de la demande: 25.11.2015
(73) Titulaire: L'Oréal, 75008 Paris (FR)
(72) Inventeur: THOMAS, Beatrice, F-95170 Deuil la Barre (FR); DRILLON, Damien, 93400 SAINT-OUEN (FR); PEMOSSO, Carine, F-75014 Paris (FR); PINAY, Frederik, F-95310 Saint Ouen l'Aumône (FR)
(74) Mandataire: L'Oreal
(86) Numéro de dépôt international: PCT/FR2014/050075
(87) Numéro de publication internationale: WO 2014/111655

(56) Documents cités:
- EP-A1- 1 106 165
- EP-A1- 1 516 914
- EP-A2- 0 078 138
- EP-A2- 1 444 975
- GB-A- 2 280 906
- US-A1- 2003 199 404
- US-A1- 2004 097 385
- US-A1- 2004 242 097

## Description

La présente invention concerne des compositions cosmétiques, notamment pour le nettoyage ou lavage des matières kératiniques, notamment des cheveux, se présentant sous forme de solide souple, ainsi qu'un procédé de traitement cosmétique les mettant en œuvre.

De nombreux produits de lavage cosmétique sont connus dans le domaine de l'hygiène capillaire. Ils sont généralement destinés à nettoyer les matières kératiniques tout en leur apportant de bonnes propriétés cosmétiques (conditionnement, hydratation, douceur, brillance).

Les produits classiques de nettoyage des matières kératiniques, tels que les shampoings, se présentent le plus souvent sous forme de liquides ou de crèmes plus ou moins visqueux.
Mais ces produits sont généralement difficiles à doser : plus ils sont liquides, plus ils ont tendance à s'échapper entre les doigts, rendant difficile leur dosage et générant du gaspillage, et/ou ont tendance à fuir hors de leur conditionnement, ce qui peut être très gênant lorsqu'ils viennent au contact des vêtements, par exemple lors de déplacements.
Pour modifier la texture, et notamment la rendre plus compacte, les moyens classiques consistent à utiliser des épaississants, mais cela se fait souvent au détriment des effets cosmétiques de la composition. En outre, on a constaté que les compositions plus épaisses présentent souvent l'inconvénient de nécessiter beaucoup d'eau de rinçage afin d'éliminer le surplus de produit sur les cheveux. Dans de nombreux pays où l'accès à l'eau est restreint, le temps de rinçage et donc la quantité nécessaire pour bien rincer le produit sont des indicateurs clés des qualités d'usage d'une composition.
Afin de surmonter certains de ces problèmes, des formulations cosmétiques solides ont été proposées, mais elles présentent généralement l'inconvénient d'être difficilement fractionnables et/ou délitables au contact de l'eau, rendant difficile leur utilisation et nécessitant également une assez grande quantité d'eau pour un emploi optimum. Par ailleurs, la rapidité de démarrage de la mousse n'est pas optimale avec ces compositions épaissies ou solides. Enfin ces produits ne laissent pas toujours un toucher naturel propre des matières kératiniques, après élimination à l'eau. En outre, les utilisateurs recherchent de plus en plus de nouvelles textures et de nouveaux concepts de produits de lavage des matières kératiniques.

EP1516914 décrit des compositions cosmétiques pour le nettoyage ou le lavage des matières kératiniques se présentant sous la forme d'un solide souple permettant d'obtenir une mousse abondante et d'apporter de bonnes propriétés cosmétiques à la peau ou aux cheveux. En particulier, certains exemples concernent des compositions aqueuses sous forme d'une pâte ou d'un solide comprenant 20% en poids de tensioactifs anioniques et des particules solides pleines telles que de la poudre de talc.

Il existe donc un besoin de disposer de compositions de lavage des matières kératiniques, qui ne coulent pas, qui soient plus compactes, modelables et économiques. Les compositions recherchées doivent être d'application aisée sur les matières kératiniques, et doivent permettre un démarrage de mousse rapide, c'est-à-dire l'obtention rapide d'une mousse adéquate et suffisamment abondante, lorsque la composition est appliquée, généralement en frottant, sur lesdites matières kératiniques éventuellement préalablement humidifiées.

La présente invention a pour but de proposer de telles compositions, ne présentant pas les inconvénients de l'art antérieur, et susceptible de permettre rapidement l'obtention d'une mousse adéquate pour le lavage des matières kératiniques.

L'invention a donc pour objet une composition cosmétique aqueuse sous forme de solide souple, comprenant :
(i) un ou plusieurs tensioactifs anioniques, en une quantité totale variant de 15 à 60% en poids par rapport au poids total de la composition,
   ladite composition comprenant au moins un tensioactif anionique choisi parmi les acyliséthionates, de préférence ayant un groupe acyle comportant de 6 à 30 atomes de carbone, mieux de 12 à 24, encore mieux de 16 à 22 atomes de carbone;
   la quantité totale en acyliséthionates dans la composition variant de 12 à 35% en poids par rapport au poids total de la composition ;
(ii) un ou plusieurs agents conditionneurs polymériques choisis parmi les polymères cationiques non siliconés, les polymères amphotères non siliconés, les silicones, notamment aminées, et leurs mélanges ;
   ladite composition comprenant au moins 20% en poids d'eau par rapport au poids total de la composition ; et
   ladite composition présentant une contrainte de seuil à 25°C supérieure ou égale à 100 Pa, et/ou présentant une force de pénétration à 25°C supérieure ou égale à 210 g, et/ou présentant un spectre viscoélastique à 25°C, mesuré entre 10⁻² Hz et 100 Hz, tel qu'il n'y a pas de point de croisement entre les courbes G' et G"; G' étant toujours strictement supérieur à G".

La composition selon l'invention présente une texture tout à fait inhabituelle, non collante et relativement ferme; elle est simple à prélever, à manipuler et à appliquer, la composition est facilement préhensible et ne s'écoule pas entre les doigts. Elle est très aisément dosable et applicable; elle ne coule pas et se rince facilement, tout en conférant un toucher naturel et propre aux matières kératiniques après élimination.

En outre, l'association selon l'invention permet d'obtenir une variété de texture de solide souple, sans ajout d'épaississant; la répartition de la composition sur les matières kératiniques est améliorée, et la rapidité de démarrage de la mousse et le temps de rinçage sont également améliorés.

Préférentiellement, la composition selon l'invention est non colorante.
Par composition non colorante, on entend selon la présente invention, une composition ne contenant pas de colorant des fibres kératiniques tels que les colorants directs ou les précurseurs de colorant d'oxydation (bases et coupleurs). S'ils sont présents, leur teneur ne dépasse pas 0,005% en poids par rapport au poids total de la composition. En effet, à une telle teneur, seule la composition serait teintée, c'est-à-dire qu'on n'observerait pas d'effet de coloration des fibres kératiniques.

Avantageusement, la composition selon l'invention est une composition moussante.

La composition selon l'invention se présente donc sous forme de solide souple. Par solide souple, on entend notamment le fait que la composition ne s'écoule pas sous son propre poids, mais peut être déformée par pression, par exemple avec un doigt; sa consistance se rapproche de celle d'un beurre (sans le caractère gras bien sur), malléable et préhensible. La composition peut être modelée aisément à la main; elle peut également être rompue facilement à la main afin de ne prélever que la quantité nécessaire de produit. En particulier, cette composition peut être conditionnée sous forme de monodose, par exemple sous forme de berlingots.

La composition solide souple selon l'invention répond à au moins l'un des critères physicochimiques ci-après, notamment au moins deux critères, préférentiellement les trois critères.
Sans indication contraire, ces critères sont mesurés à température ambiante (25°C) et pression atmosphérique (1 atm), la composition ayant subi une centrifugation de 15 minutes à 10 kg pour supprimer les bulles pour l'évaluation des critères 1 et 2.
Critère 1 : la composition selon l'invention présente un spectre viscoélastique à 25°C, mesuré entre 10⁻² Hz et 100 Hz, tel qu'il n'y a pas de point de croisement entre les courbes G' et G"; G' étant toujours strictement supérieur à G" (pour des mesures effectuées à une fréquence comprise entre 10⁻² Hz et 100 Hz).
   Le spectre viscoélastique est établi à l'aide d'un rhéomètre Thermo Haake RS600 à contrainte imposée en géométrie cône-plan. La température a été régulée par un plan à effet Peltier et un dispositif anti-évaporation (piège à solvant rempli d'eau pour les mesures à 25°C).
   On a effectué des mesures en oscillation entre 10⁻² Hz et 100 Hz, à la déformation de 0,03% avec un cône C60 1°/Ti sablé et/ou à la déformation de 0,05% avec un cône C35 2/Ti sablé.
   On mesure G' qui correspond au module de conservation traduisant la réponse élastique et le caractère solide de l'échantillon; on mesure également G" qui correspond au module de perte traduisant la réponse visqueuse et le caractère liquide de l'échantillon.
Critère 2 : la composition selon l'invention est telle qu'elle présente une contrainte de seuil à 25°C supérieure ou égale à 100 Pa.
   La contrainte de seuil est déterminée par balayage en contrainte à 25°C. On utilise un rhéomètre Thermo Haake RS600 à contrainte imposée en géométrie cône-plan sablée. La température a été régulée par un plan à effet Peltier et un dispositif anti-évaporation (piège à solvant rempli d'eau pour les mesures à 25°C).
   On effectue une rampe logarithmique de contrainte de 0,1 à 250 Pa, sur une durée de 2 minutes. Deux droites d'ajustement correspondant aux régimes stationnaires (comportements solide et liquide) sont tracées sur la courbe représentant la déformation en fonction de la contrainte (coordonnées logarithmiques). L'intersection de ces deux droites fournit la valeur recherchée.
   La composition selon l'invention est telle qu'elle présente une contrainte de seuil supérieure ou égale à 100 Pa, de préférence allant de 100 à 900 Pa, à 25°C.
Critère 3 : la composition selon l'invention est telle qu'elle présente une force de pénétration à 25°C supérieure ou égale à 210 g.
   La force de pénétration est déterminée par pénétrométrie, avec un embout de diamètre de 1,5 cm et à une vitesse de 10 mm/s. Les mesures d'analyse de texture sont effectuées à 25°C à l'aide d'un texturomètre Stable Micro Systems TA.XT Plus. Les expériences de pénétrométrie sont réalisées avec une tige métallique munie d'un embout vissé Delrin, de diamètre de 15 mm, et de hauteur de 6 mm; relié à la tête de mesure. Le piston s'enfonce dans l'échantillon à vitesse constante de 10 mm/s, sur une hauteur de 15 mm ou de 20 mm selon la hauteur de produit dans le pot (de diamètre 90 mm, et hauteur 30 mm, en plastique). On enregistre la force exercée sur le piston et la valeur moyenne de la force est calculée.
   La composition selon l'invention est telle qu'elle présente une force de pénétration à 25°C supérieure ou égale à 210 g, de préférence allant de 210 à 900 g, mieux allant de 350 à 800 g.

De préférence, la composition selon l'invention est telle qu'elle présente une contrainte de seuil à 25°C supérieure ou égale à 100 Pa, de préférence allant de 100 à 900 Pa; et/ou est telle qu'elle présente une force de pénétration à 25°C supérieure ou égale à 210 g, de préférence allant de 210 à 900 g, mieux allant de 350 à 800 g.

Encore mieux, la composition selon l'invention est telle qu'elle présente à la fois une contrainte de seuil à 25°C supérieure ou égale à 100 Pa, de préférence allant de 100 à 900 Pa; et une force de pénétration à 25°C supérieure ou égale à 210 g, de préférence allant de 210 à 900 g, mieux allant de 350 à 800 g.

Dans la présente description, l'expression "au moins un" est équivalente à l'expression "un ou plusieurs"; et l'expression "comprise entre ... et ..." est équivalente à l'expression "allant de ... à ...", ce qui sous-entend que les bornes sont inclues.

### Tensioactifs anioniques

La composition selon l'invention comprend au moins un tensioactif anionique choisi parmi les acyliséthionates ayant un groupe acyle comportant de préférence de 6 à 30 atomes de carbone, mieux de 12 à 24, encore mieux de 16 à 22 atomes de carbone.
Ils répondent de préférence à la formule suivante : R-C(O)-O-CH₂CH₂SO₃M, dans laquelle R-C(O) est un groupe acyle comportant de préférence de 6 à 30 atomes de carbone, mieux de 12 à 24, encore mieux de 16 à 22 atomes de carbone, et M désigne un contre-ion cosmétiquement acceptable.

On peut bien évidemment utiliser un mélange de tensioactifs anioniques choisis parmi les acyliséthionates.

De préférence, la composition selon l'invention comprend un ou plusieurs tensioactifs anioniques choisis parmi les cocoyl-iséthionates et/ou les lauroyliséthionates.

Les formes salifiées sont en particulier les sels de métaux alcalins tels que les sels de sodium, les sels d'ammonium, les sels d'amines, les sels d'aminoalcools ou les sels de métaux alcalino-terreux, par exemple, de magnésium. On utilise de préférence les sels de métaux alcalins ou alcalinoterreux, et en particulier les sels de sodium ou de magnésium.

La composition selon l'invention peut en outre comprendre un ou plusieurs tensioactifs anioniques additionnels.
On entend par tensioactif anionique, un tensioactif ne comportant à titre de groupements ioniques ou ionisables que des groupements anioniques. Ces groupements anioniques sont choisis de préférence parmi les groupements CO₂H, CO₂⁻, SO₃H, SO₃⁻, OSO₃H, OSO₃⁻, -H₂PO₃, -HPO₃⁻, -PO₃²⁻, -H₂PO₂, =HPO₂, -HPO₂⁻, =PO₂⁻, =POH, =PO⁻. Les tensioactifs anioniques additionnels peuvent être oxyalkylénés et comportent alors de préférence de 1 à 50 motifs oxyde d'éthylène, mieux de 1 à 10 motifs oxyde d'éthylène.
De préférence, les tensioactifs anioniques additionnels sont choisis parmi les tensioactifs anioniques sulfate(s), sulfonate(s) ou carboxylate(s), préférentiellement parmi les tensioactifs anioniques sulfonate et les tensioactifs anioniques carboxylate. Dans un mode de réalisation préféré, la composition ne comprend pas de tensioactifs additionnels, ou, si elle en comprend, ne comprend pas de tensioactifs anioniques additionnels sulfate(s) (à groupement sulfate).
Selon l'invention, on comprend dans les tensioactifs anioniques sulfonate, les tensioactifs anioniques sulfonate sans groupe carboxylate; et dans les tensioactifs anioniques carboxylate, les tensioactifs anioniques carboxylate pouvant éventuellement comprendre en outre un groupe sulfate ou sulfonate par exemple.

Les tensioactifs anioniques sulfates ou sulfonates sans groupe carboxylique pouvant être utilisés dans la composition selon l'invention à titre de tensioactifs anioniques additionnels peuvent être notamment choisis parmi les alkylsulfates, les alkyléthersulfates, les alkylamidoéthersulfates, les alkylarylpolyéthersulfates, les monoglycéride-sulfates, les alkylsulfonates, les alkylamidesulfonates, les alkylaryl-sulfonates, les alpha-oléfine-sulfonates, les paraffine-sulfonates, les N-acyltaurates, les N-méthyl, N-acyltaurates, et les formes acides correspondantes, les groupes alkyle et acyle de tous ces composés comportant de préférence de 6 à 30 atomes de carbone, mieux de 12 à 24, voire de 16 à 22 atomes de carbone, et le groupe aryle désignant de préférence un groupe phényle ou benzyle.
Les tensioactifs anioniques carboxylates susceptibles d'être utilisés dans la composition selon l'invention à titre de tensioactifs anioniques additionnels peuvent être choisis parmi les alkylsulfosuccinates, les alkyléthersulfosuccinates, les alkylamide-sulfosuccinates, les acylglycinates, les acylsarcosinates et les acylglutamates, et les formes acides correspondantes, les groupes alkyle et/ou acyle de ces composés comportant de 6 à 30 atomes de carbone, mieux de 12 à 24, voire de 16 à 22 atomes de carbone.
On peut également utiliser les monoesters d'alkyle et d'acides polyglycoside-polycarboxyliques tels que les polyglycoside-citrates d'alkyle, les polyglycoside-tartrates d'alkyle et les polyglycoside-sulfosuccinates d'alkyle, les alkylsulfosucci-namates, le groupe alkyle ou acyle de ces composés comportant de 6 à 30 atomes de carbone, mieux de 12 à 24, voire de 16 à 22 atomes de carbone; on peut également employer leurs sels.
On peut également utiliser les acyllactylates dont le groupe acyle comporte de 6 à 30 atomes de carbone, mieux de 8 à 20, voire de 12 à 24 atomes de carbone.
On peut encore citer les acides alkyl-D-galactoside-uroniques, les acides (alkyl en C14-30)éther-carboxyliques polyoxyalkylénés, les acides (alkyl en C14-30)(aryl en C6-30)éther-carboxyliques polyoxyalkylénés, les acides (alkyl en C14-30)amidoéther-carboxyliques polyoxyalkylénés; ainsi que les sels de tous ces composés; de préférence, les composés comportant de 2 à 50 motifs oxyde d'éthylène; ainsi que leurs mélanges.

De préférence, la composition comprend un ou plusieurs tensioactifs anioniques additionnels choisis parmi :
- les alkylsulfates en C6-C24, notamment en C12-C20,
- les alkyléthersulfates en C6-C24, notamment en C12-C20; comprenant de préférence de 2 à 20 motifs oxyde d'éthylène;
- les acylglutamates en C6-C24, notamment en C12-C20; notamment les stéaroylglutamates;
- les acylsarcosinates en C6-C24, notamment en C12-C20; notamment les palmitoylsarcosinates;
- les acyllactylates en C6-C24, notamment en C12-C20; notamment les béhénoyllactylates;
- les alkyl(C6-C24)éthercarboxylates, de préférence les alkyl(C12-C20)éthercarboxylates;
- les alkylsulfosuccinates en C6-C24, notamment en C12-C20, notamment les laurylsulfosuccinates.

Les formes salifiées sont en particulier les sels de métaux alcalins tels que les sels de sodium, les sels d'ammonium, les sels d'amines, les sels d'aminoalcools ou les sels de métaux alcalino-terreux, par exemple, de magnésium. On utilise de préférence les sels de métaux alcalins ou alcalinoterreux, et en particulier les sels de sodium ou de magnésium.

Préférentiellement, la composition selon l'invention comprend un ou plusieurs tensioactifs anioniques choisis parmi les acyliséthionates définis ci-dessus, associés à un ou plusieurs tensioactifs choisis parmi les alkylsulfosuccinates en C6-C24, notamment en C12-C20.

De préférence, la quantité totale de tensioactif(s) anionique(s) dans la composition de l'invention varie de 30 à 60% en poids, par rapport au poids total de la composition.
De préférence, la quantité totale de tensioactif(s) anionique(s) choisis parmi les tensioactifs anioniques sulfonate et les tensioactifs anioniques carboxylate, dans la composition selon l'invention varie de 12 à 60% en poids, mieux de 15 à 60% en poids, encore mieux de 20 à 60% en poids, préférentiellement de 25 à 50% en poids, par rapport au poids total de la composition,
De préférence, la quantité totale d'acyliséthionates dans la composition selon l'invention, varie de 15 à 35% en poids, encore mieux de 20 à 35% en poids, par rapport au poids total de la composition,

### Agents conditionneurs polymériques

La composition selon l'invention comprend au moins un agent conditionneur polymérique; elle peut bien évidemment comprendre plusieurs agents conditionneurs polymériques.
Dans le cadre de la présente demande, on entend par agent conditionneur tout agent ayant pour fonction l'amélioration des propriétés cosmétiques des cheveux, en particulier la douceur, la brillance, le démêlage, le toucher, le lissage, l'électricité statique.

Les agents conditionneurs polymériques susceptibles d'être utilisés dans le cadre de la présente invention sont de préférence choisis parmi les polymères cationiques non siliconés, les polymères amphotères non siliconés, les silicones, et leurs mélanges. Préférentiellement, ils sont choisis parmi les polymères cationiques non siliconés et les silicones, et encore mieux leurs mélanges.
1/ On entend par "polymère cationique", tout polymère comprenant des groupements cationiques et/ou des groupements ionisables en groupements cationiques. De préférence, le polymère cationique est hydrophile ou amphiphile. Les polymères cationiques peuvent être choisis parmi tous ceux déjà connus en soi comme améliorant les propriétés cosmétiques des cheveux, à savoir notamment ceux décrits dans les documents EP337354, FR2270846, FR2383660, FR2598611, FR2470596 et FR2519863.
   Les polymères cationiques préférés sont choisis parmi ceux qui contiennent des motifs comportant des groupements amines primaires, secondaires, tertiaires et/ou quaternaires pouvant soit faire partie de la chaîne principale polymère, soit être portés par un substituant latéral directement relié à celle-ci.
   Les polymères cationiques susceptibles d'être utilisés ont de préférence une masse molaire moyenne en poids (Mw) comprise entre 500 et 5.10⁶ environ, de préférence comprise entre 10³ et 3.10⁶ environ.

Parmi les polymères cationiques, on peut citer plus particulièrement les polymères du type polyamine, polyaminoamide et polyammonium quaternaire, tels que ceux décrits dans FR2505348 et FR2542997.

On peut en particulier citer :
(1) les homopolymères ou copolymères dérivés d'esters ou d'amides acryliques ou méthacryliques et comportant au moins un des motifs de formule suivante : 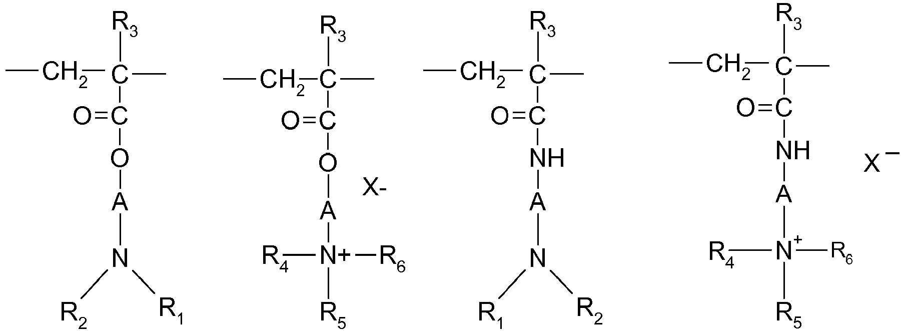 dans lesquelles:
   - R3, identiques ou différents, désignent un atome d'hydrogène ou un radical CH3;
   - A, identiques ou différents, représentent un groupe divalent alkyle, linéaire ou ramifié, de 1 à 6 atomes de carbone, de préférence 2 ou 3 atomes de carbone ou un groupe hydroxyalkyle de 1 à 4 atomes de carbone ;
   - R4, R5, R6, identiques ou différents, représentent un groupe alkyle ayant de 1 à 18 atomes de carbone ou un radical benzyle; de préférence un groupe alkyle ayant de 1 à 6 atomes de carbone;
   - R1 et R2, identiques ou différents, représentent un atome d'hydrogène ou un groupe alkyle ayant de 1 à 6 atomes de carbone, de préférence méthyle ou éthyle;
   - X désigne un anion dérivé d'un acide minéral ou organique tel qu'un anion méthosulfate ou un halogénure tel que chlorure ou bromure.

   Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques. Parmi ces copolymères de la famille (1), on peut citer :
   - les copolymères d'acrylamide et de diméthylaminoéthyl méthacrylate quaternisé au sulfate de diméthyle ou avec un halogénure de diméthyle, tels que celui vendu sous la dénomination HERCOFLOC par la société HERCULES,
   - les copolymères d'acrylamide et de chlorure de méthacryloyloxyéthyltriméthyl-ammonium, tels que ceux décrits dans EP080976 et ceux vendus sous la dénomination BINA QUAT P 100 par la société CIBA GEIGY,
   - le copolymère d'acrylamide et de méthosulfate de méthacryloyloxyéthyltriméthyl-ammonium, tel que celui vendu sous la dénomination RETEN par la société HERCULES,
   - les copolymères vinylpyrrolidone/acrylate ou méthacrylate de dialkylaminoalkyle, quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
   - les terpolymères méthacrylate de diméthylaminoéthyle/ vinylcaprolactame/ vinylpyrrolidone, tel que le produit vendu sous la dénomination GAFFIX VC 713 par la société ISP,
   - les copolymères vinylpyrrolidone/ méthacrylamidopropyldimethylamine, tels que ceux commercialisés sous la dénomination STYLEZE CC 10 par ISP;
   - les copolymères vinylpyrrolidone/ méthacrylamide de diméthylaminopropyle quaternisé, tel que le produit vendu sous la dénomination "GAFQUAT HS 100" par la société ISP,
   - les polymères, de préférence réticulés, de sels de méthacryloyloxyalkyl(C1-C4) trialkyl(C1-C4)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo- ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion comprenant 50% en poids dudit copolymère dans de l'huile minérale. Cette dispersion est commercialisée sous le nom de "SALCARE® SC 92" par la société CIBA. On peut également utiliser un homopolymère réticulé du chlorure de méthacryloyloxyéthyl triméthylammonium comprenant environ 50% en poids de l'homopolymère dans de l'huile minérale ou dans un ester liquide. Ces dispersions sont commercialisées sous les noms "SALCARE® SC 95" et "SALCARE® SC 96" par la société CIBA.
(2) Les polysaccharides cationiques, notamment les celluloses et les gommes de galactomannanes cationiques. Parmi les polysaccharides cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium quaternaire et les gommes de galactomannanes cationiques.
   Les dérivés d'éthers de cellulose comportant des groupements ammonium quaternaires sont notamment décrits dans FR1492597, et on peut citer les polymères commercialisés sous la dénomination "UCARE POLYMER JR" (JR 400 LT, JR 125, JR 30M) ou "LR" (LR 400, LR 30M) par la Société AMERCHOL. Ces polymères sont également définis dans le dictionnaire CTFA comme des ammonium quaternaires d'hydroxyéthylcellulose ayant réagi avec un époxyde substitué par un groupement triméthylammonium.
   Les copolymères de cellulose cationiques ou les dérivés de cellulose greffés avec un monomère hydrosoluble d'ammonium quaternaire, sont décrits notamment dans le brevet US4131576, et on peut citer les hydroxyalkylcelluloses, comme les hydroxyméthyl-, hydroxyéthyl- ou hydroxypropyl celluloses greffées notamment avec un sel de méthacryloyléthyl triméthylammonium, de méthacrylamidopropyl triméthylammonium, de diméthyl-diallylammonium. Les produits commercialisés répondant à cette définition sont plus particulièrement les produits vendus sous la dénomination "Celquat L 200" et "Celquat H 100" par la Société National Starch.
   Les gommes de galactomannane cationiques sont décrites plus particulièrement dans les brevets US3589578 et US4031307, et on peut citer les gommes de guar comprenant des groupements cationiques trialkylammonium. On utilise par exemple des gommes de guar modifiées par un sel (par exemple un chlorure) de 2,3-époxypropyl triméthylammonium. De tels produits sont commercialisés notamment sous les dénominations JAGUAR C13 S, JAGUAR C 15, JAGUAR C 17 ou JAGUAR C162 par la société RHODIA.
(3) les polymères constitués de motifs pipérazinyle et de radicaux divalents alkylène ou hydroxyalkylène à chaînes linéaires ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans FR2162025 et FR2280361 ;
(4) les polyaminoamides solubles dans l'eau, préparés en particulier par polycondensation d'un composé acide avec une polyamine; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé; l'agent réticulant étant utilisé dans des proportions allant de 0,025 à 0,35 mole par groupement amine du polyaminoamide; ces polyaminoamides peuvent être alcoylés ou s'ils comportent une ou plusieurs fonctions amines tertiaires, quaternisés. De tels polymères sont notamment décrits dans les brevets français 2.252.840 et 2.368.508 ;
(5) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans FR1583363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(6) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone; le rapport molaire entre le polyalkylène polyamine et l'acide dicarboxylique étant de préférence compris entre 0,8:1 et 1,4:1; le polyaminoamide en résultant étant amené à réagir avec l'épichlorhydrine dans un rapport molaire d'épichlorhydrine par rapport au groupement amine secondaire du polyaminoamide compris de préférence entre 0,5:1 et 1,8:1. De tels polymères sont notamment décrits dans US3227615 et US2961347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(7) les cyclopolymères d'alkyl diallyl amine ou de dialkyl diallyl ammonium tels que les homopolymères ou copolymères comportant comme constituant principal de la chaîne des motifs répondant aux formules (I) ou (II) : 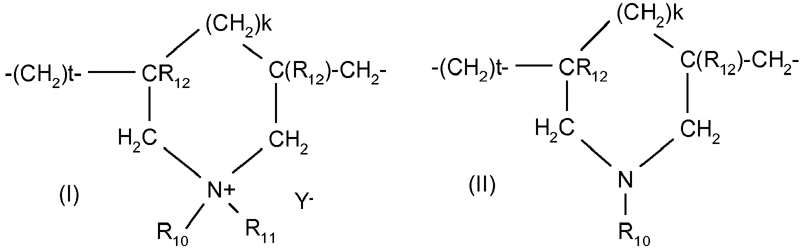 dans lesquelles
   - k et t sont égaux à 0 ou 1, la somme k + t étant égale à 1 ;
   - R12 désigne un atome d'hydrogène ou un radical méthyle ;
   - R10 et R11, indépendamment l'un de l'autre, désignent un groupement alkyle ayant de 1 à 6 atomes de carbone, un groupement hydroxyalkyle dans lequel le groupement alkyle a 1 à 5 atomes de carbone, un groupement amidoalkyle en C1-C4; ou bien R10 et R11 peuvent désigner conjointement avec l'atome d'azote auquel ils sont rattachés, des groupement hétérocycliques, tels que pipéridinyle ou morpholinyle ; R10 et R11, indépendamment l'un de l'autre, désignent de préférence un groupement alkyle ayant de 1 à 4 atomes de carbone.
   - Y⁻ est un anion tel que bromure, chlorure, acétate, borate, citrate, tartrate, bisulfate, bisulfite, sulfate, phosphate.

   Ces polymères sont notamment décrits dans FR2080759 et FR2190406. On peut citer plus particulièrement l'homopolymère de sels (par exemple chlorure) de diméthyldiallylammonium par exemple vendu sous la dénomination "MERQUAT 100" par la société NALCO (et leurs homologues de faibles masses molaires moyenne en poids) et les copolymères de sels (par exemple chlorure) de diallyldiméthylammonium et d'acrylamide commercialisés notamment sous la dénomination "MERQUAT 550" ou "MERQUAT 7SPR".
(8) les polymères de diammonium quaternaire comprenant des motifs récurrents de formule : 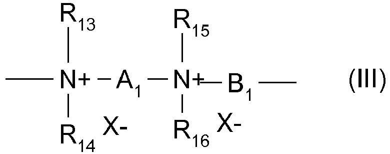 dans laquelle :
   - R13, R14, R15 et R16, identiques ou différents, représentent des radicaux aliphatiques, alicycliques, ou arylaliphatiques comprenant de 1 à 20 atomes de carbone ou des radicaux hydroxyalkylaliphatiques inférieurs, ou bien R13, R14, R15 et R16, ensemble ou séparément, constituent avec les atomes d'azote auxquels ils sont rattachés des hétérocycles comprenant éventuellement un second hétéroatome autre que l'azote ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un alkylène et D un groupement ammonium quaternaire ;
   - A1 et B1 représentent des groupements divalents polyméthyléniques comprenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   - X⁻ désigne un anion dérivé d'un acide minéral ou organique;
      Etant entendu que A1, R13 et R15 peuvent former avec les deux atomes d'azote auxquels ils sont rattachés un cycle pipérazinique ;
      en outre si A1 désigne un radical alkylène ou hydroxyalkylène linéaire ou ramifié, saturé ou insaturé, B1 peut également désigner un groupement (CH2)n-CO-D-OC-(CH2)n- dans lequel D désigne :
      a) un reste de glycol de formule -O-Z-O-, où Z désigne un radical hydrocarboné linéaire ou ramifié ou un groupement répondant à l'une des formules suivantes: -(CH2-CH2-O)x-CH2-CH2- et -[CH2-CH(CH3)-O]y-CH2-CH(CH3)- où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical divalent -CH2-CH2-S-S-CH2-CH2- ;
      d) un groupement uréylène de formule : -NH-CO-NH- ;

   De préférence, X⁻ est un anion tel que le chlorure ou le bromure.
   Ces polymères ont une masse molaire moyenne en nombre (Mn) généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020. On peut citer plus particulièrement les polymères qui sont constitués de motifs récurrents répondant à la formule : 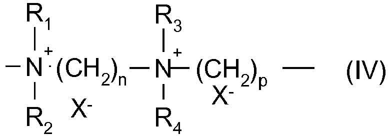 dans laquelle R1, R2, R3 et R4, identiques ou différents, désignent un radical alkyle ou hydroxyalkyle ayant de 1 à 4 atomes de carbone environ, n et p sont des nombres entiers variant de 2 à 20 environ et, X- est un anion dérivé d'un acide minéral ou organique.
   Un composé de formule (IV) particulièrement préféré est celui pour lequel R1, R2, R3 et R4 représentent un radical méthyle, n=3, p=6 et X = CI, dénommé Hexadimethrine chloride selon la nomenclature INCI (CTFA).
(9) les polymères de polyammonium quaternaires comprenant des motifs de formule (V): 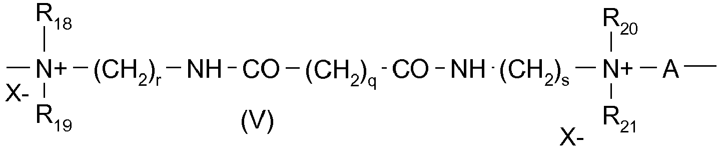 dans laquelle :
   - R18, R19, R20 et R21, identiques ou différents, représentent un atome d'hydrogène ou un radical méthyle, éthyle, propyle, β-hydroxyéthyle, β-hydroxypropyle ou
   - CH2CH2(OCH2CH2)pOH, où p est égal à 0 ou à un nombre entier compris entre 1 et 6, sous réserve que R18, R19, R20 et R21 ne représentent pas simultanément un atome d'hydrogène,
   - r et s, identiques ou différents, sont des nombres entiers compris entre 1 et 6,
   - q est égal à 0 ou à un nombre entier compris entre 1 et 34,
   - X- désigne un anion tel qu'un halogénure,
   - A désigne un radical d'un dihalogénure ou représente de préférence -CH2-CH2-O-CH2-CH2-.

   De tels composés sont notamment décrits dans la demande de brevet EP-A-122 324. On peut par exemple citer les produits "Mirapol® A 15", "Mirapol® AD1", "Mirapol® AZ1" et "Mirapol® 175" vendus par la société Miranol.
(10) Les polymères quaternaires de vinylpyrrolidone et de vinylimidazole tels que par exemple les produits commercialisés sous les dénominations Luviquat® FC 905, FC 550 et FC 370 par la société B.A.S.F.
(11) Les polyamines comme le Polyquart® H vendu par COGNIS, référencé sous le nom de "POLYETHYLENEGLYCOL (15) TALLOW POLYAMINE" dans le dictionnaire CTFA.
(12) les polymères comportant dans leur structure :
   (a) un ou plusieurs motifs répondant à la formule (A) suivante : 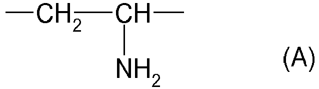
   (b) éventuellement un ou plusieurs motifs répondant à la formule (B) suivante :

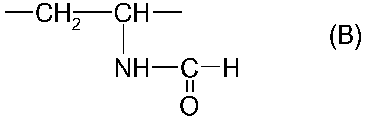

Autrement dit, ces polymères peuvent être notamment choisis parmi les homo- ou copolymères comportant un ou plusieurs motifs issus de la vinylamine et éventuellement un ou plusieurs motifs issus du vinylformamide.
De préférence, ces polymères cationiques sont choisis parmi les polymères comportant, dans leur structure, de 5 à 100% en moles de motifs répondant à la formule (A) et de 0 à 95% en moles de motifs répondant à la formule (B), préférentiellement de 10 à 100% en moles de motifs répondant à la formule (A) et de 0 à 90% en moles de motifs répondant à la formule (B).
Ces polymères peuvent être obtenus par exemple par hydrolyse partielle du polyvinylformamide. Cette hydrolyse peut se faire en milieu acide ou basique.
La masse moléculaire moyenne en poids dudit polymère, mesurée par diffraction de la lumière, peut varier de 1000 à 3.000.000 g/mole, de préférence de 10 000 à 1.000.000 et plus particulièrement de 100 000 à 500.000 g/mole.
La densité de charge cationique de ces polymères peut varier de 2 meq/g à 20 meq/g, de préférence de 2,5 à 15 et plus particulièrement de 3,5 à 10 meq/g.
Les polymères comportant des motifs de formule (A) et éventuellement des motifs de formule (B) sont notamment vendus sous la dénomination LUPAMIN par la société BASF, tels que par exemple, et de manière non limitative, les produits proposés sous la dénomination LUPAMIN 9095, LUPAMIN 5095, LUPAMIN 1095, LUPAMIN 9030 et LUPAMIN 9010.

D'autres polymères cationiques utilisables dans le cadre de l'invention sont des protéines cationiques ou des hydrolysats de protéines cationiques, des polyalkylèneimines, en particulier des polyéthylèneimines, des polymères comprenant des motifs vinylpyridine ou vinylpyridinium, des condensats de polyamines et d'épichlorhydrine, des polyuréylènes quaternaires et les dérivés de la chitine.

De préférence, les polymères cationiques non siliconés sont choisis parmi ceux des familles (1), (2), (7) et (10) ci-dessus citées, et tout particulièrement parmi ceux de la famille (2).

Parmi les polymères cationiques mentionnés ci-dessus, on peut utiliser de préférence les polysaccharides cationiques, notamment les celluloses et les gommes de galactomannanes cationiques, et en particulier les dérivés d'éther de cellulose quaternaires tels que les produits vendus sous la dénomination "JR 400" par la Société AMERCHOL, les cyclopolymères cationiques, en particulier les homopolymères ou copolymères de sels (par exemple chlorure) de diméthyldiallylammonium, vendus sous les dénominations MERQUAT 100, MERQUAT 550 et MERQUAT S par la société NALCO et leurs homologues de faibles poids moléculaires en poids, les polymères quaternaires de vinylpyrrolidone et de vinylimidazole, les homopolymères ou copolymères éventuellement réticulés de sels de méthacryloyloxyalkyl(C1-C4) trialkyl(C1-C4)ammonium; et leurs mélanges.

2/ Les polymères amphotères non siliconés peuvent de préférence être choisis parmi les polymères amphotères comprenant la répétition de :
(i) un ou plusieurs motifs issus d'un monomère de type (méth)acrylamide,
(ii) un ou plusieurs motifs issus d'un monomère de type (méth)acrylamidoalkyltrialkylammonium, et
(iii) un ou plusieurs motifs issus d'un monomère acide de type acide (méth)acrylique.

De préférence, les motifs issus d'un monomère de type (i) (méth)acrylamide sont des motifs de structure (I) suivante : 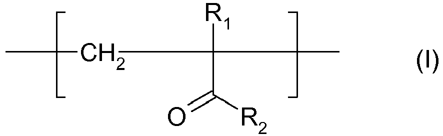 dans laquelle R₁ désigne H ou CH₃, et R₂ est choisi parmi un radical amino, diméthylamino, tert-butylamino, dodécylamino, ou -NH-CH₂OH.
De préférence, ledit polymère amphotère ne comprend la répétition que d'un seul motif de formule (I).
Le motif issu d'un monomère de type (méth)acrylamide de formule (I) dans laquelle R₁ désigne H et R₂ est un radical amino (NH2) est particulièrement préféré. Il correspond au monomère acrylamide proprement dit.

De préférence, les motifs issus d'un monomère de type (ii) (méth)acrylamidoalkyltrialkylammonium sont des motifs de structure (II) suivante : 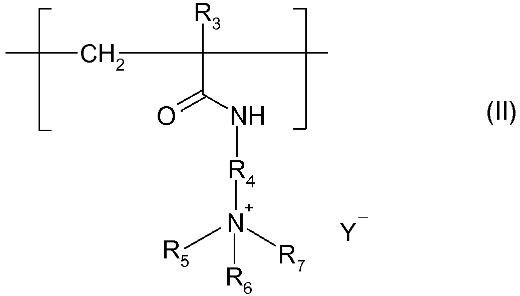 dans laquelle :
- R₃ désigne H ou CH₃,
- R₄ désigne un groupement (CH₂)k avec k un nombre entier allant de 1 à 6, et de préférence de 2 à 4 ;
- R₅, R₆ et R₇, identiques ou différents, désignent chacun un groupement alkyle ayant de 1 à 4 atomes de carbone;
- Y⁻ est un anion tel que bromure, chlorure, acétate, borate, citrate, tartrate, bisulfate, bisulfite, sulfate, phosphate.

De préférence, ledit polymère amphotère ne comprend la répétition que d'un seul motif de formule (II).
Parmi ces motifs issus d'un monomère de type (méth)acrylamidoalkyltrialkylammonium de formule (II), on préfère ceux issus du monomère chlorure de méthacrylamidopropyltriméthylammonium, pour lequel R₃ désigne un radical méthyle, k vaut 3, R₅, R₆ et R₇ désignent un radical méthyle, et Y⁻ désigne un anion chlorure.

De préférence, les motifs issus d'un monomère de type (iii) acide (méth)acrylique sont des motifs de formule (III) : 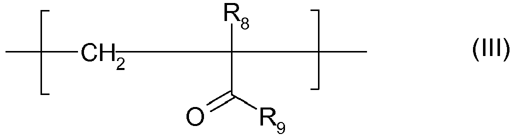 dans laquelle R₈ désigne H ou CH₃, et R₉ désigne un radical hydroxyle ou un radical -NH-C(CH₃)₂-CH₂-SO₃H.
Les motifs préférés de formules (III) correspondent aux monomères acide acrylique, acide méthacrylique et acide 2-acrylamino 2-méthyl propane sulfonique.
De préférence, le motif issu d'un monomère de type acide (méth)acrylique de formule (III) est celui issu de l'acide acrylique, pour lequel R₈ désigne un atome d'hydrogène et R₉ désigne un radical hydroxyle.
Le ou les monomères acides de type acide (méth)acrylique peuvent être non neutralisés, ou partiellement ou totalement neutralisés par une base organique ou minérale.
De préférence, ledit polymère amphotère ne comprend la répétition que d'un seul motif de formule (III).

Selon un mode de réalisation préféré de l'invention, le ou les polymères amphotères de ce type comprennent au moins 30% en mole de motifs issus d'un monomère de type (i) (méth)acrylamide. De préférence, ils comprennent de 30 à 70% en mole de motifs issus d'un monomère de type (méth)acrylamide, de manière plus préférée de 40 à 60% en mole.
La teneur en motifs issus d'un monomère de type (ii) (méth)acrylamido alkyltrialkylammonium peut avantageusement être de 10 à 60%, préférentiellement de 20 à 55% en moles.
La teneur en motifs issus d'un monomère acide de type (iii) acide (méth)acrylique peut avantageusement être de 1 à 20%, préférentiellement de 5 à 15% en moles. Selon un mode de réalisation particulièrement préféré de l'invention, le polymère amphotère de ce type comprend :
- de 30 à 70% en moles de motifs issus d'un monomère de type (i) (méth)acrylamide, de manière plus préférée de 40 à 60% en moles,
- de 10 à 60% en moles, préférentiellement de 20 à 55% en moles, de motifs issus d'un monomère de type (ii) (méth)acrylamidoalkyltrialkylammonium, et
- de 1 à 20% en moles, préférentiellement de 5 à 15% en moles, de motifs issus d'un monomère de type (iii) acide (méth)acrylique.

Ce type de polymères amphotères peut également comprendre des motifs additionnels, différents des motifs issus d'un monomère de type (méth)acrylamide, de type (méth)acrylamidoalkyltrialkylammonium et de type acide (méth)acrylique tels que décrits ci-avant.
Toutefois, selon un mode de réalisation préféré de l'invention, lesdits polymères amphotères sont constitués uniquement de motifs issus de monomères de type (i) (méth)acrylamide, de type (ii) (méth)acrylamidoalkyltrialkylammonium et de type (iii) acide (méth)acrylique.
Comme exemple de polymères amphotères particulièrement préférés, on peut citer les terpolymères acrylamide/chlorure de méthacrylamidopropyltriméthylammonium/acide acrylique. De tels polymères sont répertoriés dans le dictionnaire C.T.F.A. International Cosmetic Ingredient Dictionary, 10ème édition 2004, sous la dénomination "Polyquaternium 53". Des produits correspondants sont notamment commercialisés sous les dénominations MERQUAT 2003 et MERQUAT 2003 PR par la société NALCO.

Comme autre type de polymère amphotère susceptible d'être utilisé, on peut également citer les copolymères à base d'acide (méth)acrylique et d'un sel de dialkyldiallylammonium, tels que les copolymères d'acide (méth)acrylique et de chlorure de diméthyldiallyl ammonium. On peut citer par exemple le Merquat 280 proposé par la société NALCO.

3/ Les silicones susceptibles d'être employées peuvent être choisies parmi les polydialkylsiloxanes, notamment les polydiméthylsiloxanes (PDMS), et les polysiloxanes organomodifiés comportant au moins un groupement fonctionnel choisi de préférence parmi les groupements aryle, les groupements aminés, les groupements alcoxy et les groupements polyoxyéthylénés, ou polyoxypropylénés.
Les silicones sont notamment définies plus en détail dans l'ouvrage de Walter NOLL "Chemistry and Technology of Silicones" (1968), Academie Press. Elles peuvent être volatiles ou non volatiles, et se présenter sous forme d'huile, de gomme ou de résine; les huiles et les gommes de silicone sont préférées.

Lorsqu'elles sont volatiles, les silicones peuvent être plus particulièrement choisies parmi celles possédant un point d'ébullition compris entre 60°C et 260°C, et plus particulièrement encore parmi:
(i) les polydialkylsiloxanes cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE® 7207 par UNION CARBIDE ou SILBIONE® 70045 V2 par RHODIA, le décaméthylcyclopentasiloxane commercialisé sous le nom de VOLATILE SILICONE® 7158 par UNION CARBIDE, et SILBIONE® 70045 V5 par RHODIA, ainsi que leurs mélanges.
   On peut également citer les cyclocopolymères du type diméthylsiloxanes/ méthylalkylsiloxane, tel que la SILICONE VOLATILE® FZ 3109 commercialisée par la société UNION CARBIDE, de formule : 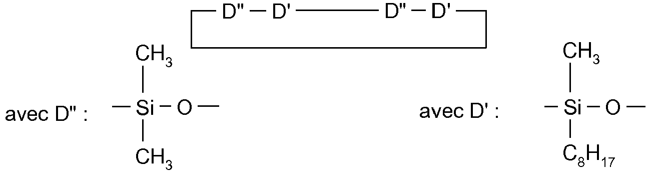 On peut également citer les mélanges de polydialkylsiloxanes cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néo pentane;
(ii) les polydialkylsiloxanes volatiles linéaires ayant 2 à 9 atomes de silicium et présentant une viscosité inférieure ou égale à 5.10⁻⁶ m²/s à 25°C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile silicone fluids for cosmetics" .
   On utilise de préférence des polydialkylsiloxanes non volatiles, des polyorganosiloxanes modifiés par les groupements organofonctionnels ci-dessus ainsi que leurs mélanges. Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.
   Parmi ces polydialkylsiloxanes, on peut citer les produits commerciaux suivants :
   - les huiles SILBIONE® des séries 47 et 70 047 ou les huiles MIRASIL® commercialisées par RHODIA telles que, par exemple l'huile 70 047 V 500 000;
   - les huiles de la série MIRASIL® commercialisées par la société RHODIA;
   - les huiles de la série 200 de la société DOW CORNING telles que la DC200 ayant viscosité 60 000 mm²/s ;
   - les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol connus sous le nom de dimethiconol (CTFA), tels que les huiles de la série 48 de la société RHODIA.
Dans cette classe de polydialkylsiloxanes, on peut également citer les produits commercialisés sous les dénominations "ABIL WAX® 9800 et 9801" par la société GOLDSCHMIDT qui sont des polydialkyl (C1-C20) siloxanes.
Des produits plus particulièrement utilisables conformément à l'invention sont des mélanges tels que :
- les mélanges formés à partir d'un polydiméthylsiloxane hydroxylé en bout de chaîne, ou dimethiconol (CTFA) et d'un polydiméthylsiloxane cyclique également appelé cyclométhicone (CTFA) tel que le produit Q2-1401 commercialisé par la société DOW CORNING.

Les silicones organomodifiées susceptibles d'être utilisées dans la présente invention sont notamment des silicones telles que définies précédemment et comportant dans leur structure un ou plusieurs groupements organofonctionnels fixés par l'intermédiaire d'un groupe hydrocarboné.
Les silicones organomodifiées peuvent être des polydiarylsiloxanes, notamment des polydiphénylsiloxanes, et des polyalkylarylsiloxanes fonctionnalisés par les groupes organofonctionnels mentionnés précédemment.
Les polyalkylarylsiloxanes sont particulièrement choisis parmi les polydiméthyl/méthylphénylsiloxanes, les polydiméthyl /diphénylsiloxanes linéaires et/ou ramifiés de viscosité allant de 1.10⁻⁵ à 5.10⁻²m²/S à 25°C.
Parmi ces polyalkylarylsiloxanes, on peut citer les produits commercialisés sous les dénominations suivantes :
- les huiles SILBIONE® de la série 70 641 de RHODIA;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.
Parmi les silicones organomodifiées, on peut aussi citer les polyorganosiloxanes comportant :
- des groupements aminés substitués ou non comme les produits commercialisés sous la dénomination GP 4 Silicone Fluid et GP 7100 par la société GENESEE Les groupements aminés substitués sont en particulier des groupements aminoalkyle en C1-C4 ;
- des groupements alcoxylés, comme le produit commercialisé sous la dénomination "SILICONE COPOLYMER F-755" par SWS SILICONES;
- des groupements oxyéthylénés ou oxypropylénés.

Dans une variante de l'invention, les silicones ne sont pas organomodifiées.
De préférence, les silicones sont cationiques ou non ioniques.

Les silicones employées peuvent également être choisies parmi les silicones aminées, et notamment peuvent répondre à la formule suivante : 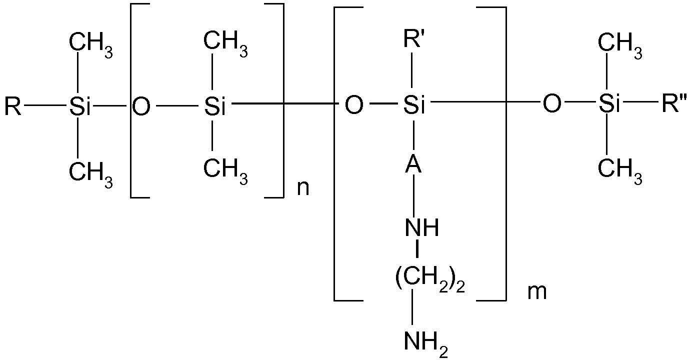 dans laquelle R, R', R", identiques ou différents, désignent un radical alkyle en C₁-C₄, de préférence CH₃; un radical alcoxy en C₁-C₄, de préférence méthoxy; ou OH ; A représente un radical alkylène, linéaire ou ramifié, en C1-C8, de préférence en C₃-C₆; m et n sont des nombres entiers dépendant du poids moléculaire et dont la somme est comprise entre 1 et 2000.

Selon une première possibilité, R, R', R", identiques ou différents, représentent un radical alkyle en C₁-C₄ de préférence méthyleou hydroxyle, A représente un radical alkylène en C₁-C₈ de préférence en C₃-C₄ et m et n sont tels que la masse moléculaire moyenne en poids du composé est comprise entre 5 000 et 500 000 environ. Les composés de ce type sont dénommés dans le dictionnaire CTFA, "amodiméthicone".
Selon une deuxième possibilité, R, R', R", identiques ou différents, représentent un radical alcoxy en C₁-C₄ ou hydroxyle, l'un au moins des radicaux R ou R" est un radical alcoxy et A représente un radical alkylène en C₃. Le rapport molaire hydroxy / alcoxy est de préférence compris entre 0,2/1 et 0,4/1 et avantageusement égal à 0,3/1. Par ailleurs, m et n sont tels que la masse moléculaire moyenne en poids du composé est comprise entre 2000 et 10⁶. Plus particulièrement, n est compris entre 0 et 999 et m est compris entre 1 et 1000, la somme de n et m étant comprise entre 1 et 1000. Dans cette catégorie de composés, on peut citer entre autres, le produit Belsil®ADM 652, commercialisée par Wacker.
Selon une troisième possibilité, R, R", différents, représentent un radical alcoxy en C₁-C₄ ou hydroxyle, l'un au moins des radicaux R, R" est un radical alcoxy, R' représente un radical méthyle et A représente un radical alkylène en C₃. Le rapport molaire hydroxy / alcoxy est de préférence compris entre 1/0,8 et 1/1,1, et avantageusement est égal à 1/0,95. Par ailleurs, m et n sont tels que la masse moléculaire moyenne en poids du composé est comprise entre 2000 et 200000. Plus particulièrement, n est compris entre 0 et 999 et m est compris entre 1 et 1000, la somme de n et m étant comprise entre 1 et 1000. Plus particulièrement, on peut citer le produit FluidWR® 1300, commercialisé par Wacker.

De préférence, la silicone aminée présente une masse moléculaire moyenne en poids allant de 75000 à 1000000, préférentiellement de 100000 à 200000. Les masses moléculaires moyennes en poids de ces silicones aminées sont mesurées par Chromatographie par Perméation de Gel (GPC) à température ambiante en équivalent polystyrène. Les colonnes utilisées sont des colonnes µ styragel. L'éluant est le THF, le débit est de 1 ml/mn. On injecte 200 µl d'une solution à 0,5% en poids de silicone dans le THF. La détection se fait par réfractométrie et UVmétrie.

Préférentiellement, la composition selon l'invention comprend un ou plusieurs agents conditionneurs polymériques choisis parmi les silicones notamment aminées et les polymères cationiques non siliconés, de préférence choisis parmi ceux des familles (1), (2), (7) et (10) ci-dessus citées, et tout particulièrement parmi ceux de la famille (2); ainsi que leurs mélanges.

Les agents conditionneurs polymériques sont de préférence présents dans la composition selon l'invention en une quantité allant de 0,001 à 20% en poids, préférentiellement de 0,1 à 10% en poids, mieux encore de 0,2 à 8% en poids, voire de 0,4 à 5% en poids, préférentiellement de 0,5 à 5% en poids, par rapport au poids total de la composition.

### Agents conditionneurs non polymériques

La composition selon l'invention peut en outre comprendre un agent conditionneur non polymérique; elle peut bien évidemment comprendre plusieurs agents conditionneurs non polymériques additionnels.

Les agents conditionneurs non polymériques susceptibles d'être utilisés dans le cadre de la présente invention sont de préférence choisis parmi les tensioactifs cationiques, les esters gras autres que les triglycérides, les alcools gras, les huiles végétales, les hydrocarbures liquides en C6-C16, les hydrocarbures ayant plus de 16 atomes de carbone, les céramides, les cires végétales ou animales, et leurs mélanges
1/ Le ou les tensioactifs cationiques susceptibles d'être utilisés selon l'invention peuvent être par exemple des sels d'amines grasses primaire, secondaire ou tertiaire, éventuellement polyoxyalkylénées; des sels d'ammonium quaternaire, et leurs mélanges.
   Comme sels d'ammonium quaternaire, on peut notamment citer :
   - ceux répondant à la formule générale (I) suivante : 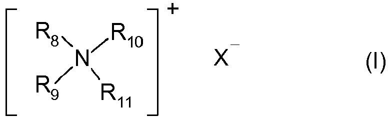 dans laquelle les groupes R₈ à R₁₁, identiques ou différents, représentent un groupe aliphatique, linéaire ou ramifié, comportant de 1 à 30 atomes de carbone, ou un groupe aromatique tel que aryle ou alkylaryle, au moins un des groupes R₈ à R₁₁ comportant de 8 à 30 atomes de carbone, de préférence de 12 à 24 atomes de carbone.
      Les groupes aliphatiques peuvent comporter des hétéroatomes tels que notamment l'oxygène, l'azote, le soufre et les halogènes. Les groupes aliphatiques sont par exemple choisis parmi les groupes alkyle en C₁-C₃₀, alcoxy en C₁-C₃₀, polyoxyalkylène (C₂-C₆), alkylamide en C₁-C₃₀, alkyl(C₁₂-C₂₂)amidoalkyle(C₂-C₆), alkyl(C₁₂-C₂₂)acétate, et hydroxyalkyle en C₁-C₃₀, X⁻ est un anion choisi dans le groupe des halogénures, phosphates, acétates, lactates, alkyl(C₁-C₄)sulfates, alkyl(C₁-C₄)- ou alkyl(C₁-C₄)arylsulfonates.
      Parmi les sels d'ammonium quaternaire de formule (I), on préfère d'une part, les chlorures de tétraalkylammonium comme, par exemple, les chlorures de dialkyldiméthylammonium ou d'alkyltriméthylammonium dans lesquels le groupe alkyle comporte environ de 12 à 22 atomes de carbone, en particulier les chlorures de béhényltriméthylammonium, de distéaryldiméthylammonium, de cétyltriméthylammonium, de benzyldiméthylstéarylammonium ou d'autre part, le chlorure de palmitylamidopropyltriméthylammonium ou le chlorure de stéaramidopropyldiméthyl-(myristyl acétate)-ammonium notamment commercialisé sous la dénomination CERAPHYL® 70 par la société VAN DYK.
   - les sels d'ammonium quaternaire de l'imidazoline, comme par exemple ceux de formule (II) suivante : 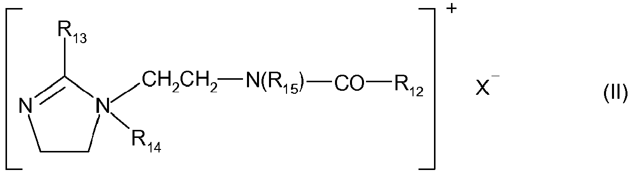 dans laquelle R₁₂ représente un groupe alcényle ou alkyle comportant de 8 à 30 atomes de carbone, par exemple dérivés des acides gras du suif, R₁₃ représente un atome d'hydrogène, un groupe alkyle en C₁-C₄ ou un groupe alcényle ou alkyle comportant de 8 à 30 atomes de carbone, R₁₄ représente un groupe alkyle en C₁-C₄, R₁₅ représente un atome d'hydrogène, un groupe alkyle en C₁-C₄, X⁻ est un anion choisi dans le groupe des halogénures, phosphates, acétates, lactates, alkyl(C₁-C₄)sulfates, alkyl(C₁-C₄)- ou alkyl(C₁-C₄)arylsulfonates.
      De préférence, R₁₂ et R₁₃ désignent un mélange de groupes alcényle ou alkyle comportant de 12 à 21 atomes de carbone, par exemple dérivés des acides gras du suif, R₁₄ désigne un groupe méthyle, R₁₅ désigne un atome d'hydrogène. Un tel produit est par exemple commercialisé sous la dénomination REWOQUAT® W 75 par la société REWO,
   - les sels de di- ou de triammonium quaternaire en particulier de formule (III) suivante : 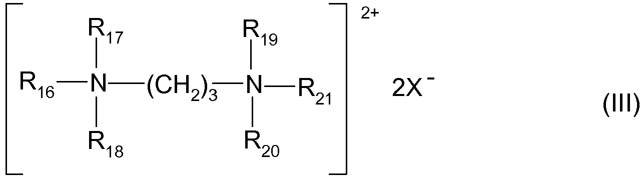 dans laquelle R₁₆ désigne un groupe alkyle comportant environ de 16 à 30 atomes de carbone éventuellement hydroxylé et/ou interrompu par un ou plusieurs atomes d'oxygène, R₁₇ est choisi parmi l'hydrogène ou un groupe alkyle comportant de 1 à 4 atomes de carbone ou un groupe -(CH₂)₃-N⁺(R₁₆ₐ)(R₁₇ₐ)(R₁₈ₐ), R₁₆ₐ, R₁₇ₐ, R₁₈ₐ, R₁₈, R₁₉, R₂₀ et R₂₁, identiques ou différents, sont choisis parmi l'hydrogène ou un groupe alkyle comportant de 1 à 4 atomes de carbone, et X⁻ est un anion choisi dans le groupe des halogénures, acétates, phosphates, nitrates, alkyl(C₁-C₄)sulfates, alkyl(C₁-C₄)- ou alkyl(C₁-C₄)arylsulfonates, en particulier méthylsulfate et éthylsulfate.
      De tels composés sont par exemple le Finquat CT-P proposé par la société FINETEX (Quaternium 89), le Finquat CT proposé par la société FINETEX (Quaternium 75),
   - les sels d'ammonium quaternaire contenant une ou plusieurs fonctions esters, tels que, par exemple, ceux de formule (IV) suivante : 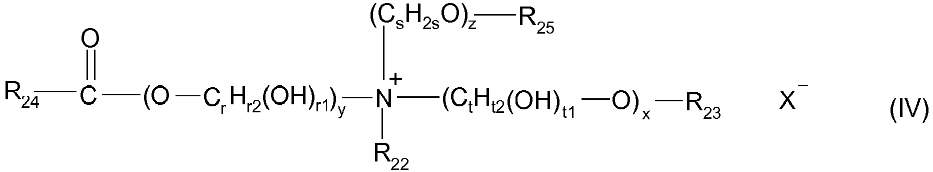 dans laquelle :
      R₂₂ est choisi parmi les groupes alkyles en C₁-C₆ et les groupes hydroxyalkyle ou dihydroxyalkyle en C₁-C₆,
      R₂₃ est choisi parmi le groupe R₂₆-C(=O)-; les groupes R₂₇ hydrocarbonés en C₁-C₂₂, linéaires ou ramifiés, saturés ou insaturés; et l'atome d'hydrogène,
      R₂₅ est choisi parmi le groupe R₂₈-C(=O)-; les groupes R₂₉ hydrocarbonés en C₁-C₆, linéaires ou ramifiés, saturés ou insaturés; et l'atome d'hydrogène,
      R₂₄, R₂₆ et R₂₈, identiques ou différents, sont choisis parmi les groupes hydrocarbonés en C₇-C₂₁, linéaires ou ramifiés, saturés ou insaturés,
      r, s et t, identiques ou différents, sont des entiers valant de 2 à 6,
      r1 et t1, identiques ou différents, valent 0 ou 1,
      r2 + r1 = 2 r et t1 + t2 = 2 t,
      y est un entier valent de 1 à 10,
      x et z, identiques ou différents, sont des entiers valant de 0 à 10,
      X⁻ est un anion simple ou complexe, organique ou inorganique,
      sous réserve que la somme x + y + z vaut de 1 à 15, que lorsque x vaut 0 alors R₂₃ désigne R₂₇ et que lorsque z vaut 0 alors R₂₅ désigne R₂₉.

Les groupes alkyles R₂₂ peuvent être linéaires ou ramifiés et plus particulièrement linéaires. De préférence, R₂₂ désigne un groupe méthyle, éthyle, hydroxyéthyle ou dihydroxypropyle, et plus particulièrement un groupe méthyle ou éthyle.
Avantageusement, la somme x + y + z vaut de 1 à 10.
Lorsque R₂₃ est un groupe R₂₇ hydrocarboné, il peut être long et avoir de 12 à 22 atomes de carbone, ou court et avoir de 1 à 3 atomes de carbone.
Lorsque R₂₅ est un groupe R₂₉ hydrocarboné, il a de préférence 1 à 3 atomes de carbone.
Avantageusement, R₂₄, R₂₆ et R₂₈, identiques ou différents, sont choisis parmi les groupes hydrocarbonés en C₁₁-C₂₁, linéaires ou ramifiés, saturés ou insaturés, et plus particulièrement parmi les groupes alkyle et alcényle en C₁₁-C₂₁, linéaires ou ramifiés, saturés ou insaturés.
De préférence, x et z, identiques ou différents, valent 0 ou 1. Avantageusement, y est égal à 1. De préférence, r, s et t, identiques ou différents, valent 2 ou 3, et encore plus particulièrement sont égaux à 2.
L'anion X⁻ est de préférence un halogénure, de préférence chlorure, bromure ou iodure, un alkyl(C₁-C₄)sulfate, alkyl(C₁-C₄)- ou alkyl(C₁-C₄)aryl-sulfonate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.
L'anion X⁻ est encore plus particulièrement le chlorure, le méthylsulfate ou l'éthylsulfate.
On utilise plus particulièrement dans la composition selon l'invention, les sels d'ammonium de formule (IV) dans laquelle :
- R₂₂ désigne un groupe méthyle ou éthyle,
- x et y sont égaux à 1,
- z est égal à 0 ou 1,
- r, s et t sont égaux à 2,
- R₂₃ est choisi parmi le groupe R₂₆-C(=O)-; les groupes méthyle, éthyle ou hydrocarbonés en C₁₄-C₂₂, l'atome d'hydrogène,
- R₂₅ est choisi parmi le groupe R₂₈-C(=O)-, l'atome d'hydrogène,
- R₂₄, R₂₆ et R₂₈, identiques ou différents, sont choisis parmi les groupes hydrocarbonés en C₁₃-C₁₇, linéaires ou ramifiés, saturés ou insaturés, et de préférence parmi les groupes alkyle et alcényle en C₁₃-C₁₇, linéaires ou ramifiés, saturés ou insaturés.
Avantageusement, les groupes hydrocarbonés sont linéaires.

On peut citer par exemple parmi les composés de formule (IV) les sels, notamment le chlorure ou le méthylsulfate de diacyloxyéthyldiméthylammonium, de diacyloxyéthyl-hydroxyéthylméthylammonium, de monoacyloxyéthyldihydroxy éthylméthylammonium, de triacyloxyéthylméthylammonium, de monoacyloxyéthylhydroxyéthyldiméthylammonium, et leurs mélanges. Les groupes acyles ont de préférence 14 à 18 atomes de carbone et proviennent plus particulièrement d'une huile végétale comme l'huile de palme ou de tournesol. Lorsque le composé contient plusieurs groupes acyles, ces derniers peuvent être identiques ou différents. Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle, de préférence de méthyle ou d'éthyle, un sulfate de dialkyle, de préférence de méthyle ou d'éthyle, le méthanesulfonate de méthyle, le para-toluènesulfonate de méthyle, la chlorhydrine du glycol ou du glycérol. De tels composés sont par exemple commercialisés sous les dénominations DEHYQUART® par la société HENKEL, STEPANQUAT® par la société STEPAN, NOXAMIUM® par la société CECA, REWOQUAT® WE 18 par la société REWO-WITCO.
La composition selon l'invention peut contenir par exemple un mélange de sels de mono-, di- et triester d'ammonium quaternaire avec une majorité en poids de sels de diester. On peut aussi utiliser les sels d'ammonium contenant au moins une fonction ester décrits dans les brevets US-A-4874554 et US-A-4137180. On peut également utiliser le chlorure de béhénoylhydroxypropyltriméthylammonium, par exemple, proposé par la société KAO sous la dénomination Quartamin BTC 131. De préférence, les sels d'ammonium contenant au moins une fonction ester contiennent deux fonctions esters.

Les tensioactifs cationiques présents dans la composition selon l'invention sont de préférence choisis parmi les sels de cétyltriméthylammonium, de béhényltriméthylammonium, de dipalmitoyléthylhydroxyéthylméthylammonium et leurs mélanges; et plus particulièrement parmi le chlorure de béhényltriméthylammonium, le chlorure de cétyltriméthylammonium, le méthosulfate de dipalmitoyléthylhydroxyéthylammonium et leurs mélanges.

2/ Les esters gras autres que les triglycérides peuvent être choisis parmi les esters gras solides à température ambiante et à pression atmosphérique (25°C, 1 atm) et les esters gras liquides, ainsi que leurs mélanges.

De préférences, les esters gras solides sont des esters d'acides carboxyliques saturés comportant au moins 10 atomes de carbone, et de monoalcools gras saturés comportant au moins 10 atomes de carbone. Les acides ou les monoalcools saturés peuvent être linéaires ou ramifiés. Les acides carboxyliques saturés comportent de préférence de 10 à 30 atomes de carbone et plus particulièrement de 12 à 24 atomes de carbone. Ils peuvent être éventuellement hydroxylés. Les monoalcools gras saturés comportent de préférence de 10 à 30 atomes de carbone et plus particulièrement de 12 à 24 atomes de carbone.
De préférence, les esters gras solides sont choisis parmi les myristates de myristyle, de cétyle et de stéaryle, les palmitates de myristyle, de cétyle et de stéaryle, les stéarates de myristyle, de cétyle et de stéaryle, et leurs mélanges.

Les esters gras liquides peuvent être des esters de monoalcools ou de polyols avec des mono ou des polyacides, au moins un des alcools et/ou des acides comportant au moins une chaîne de plus de 7 atomes de carbones. De préférence, l'ester gras liquide selon l'invention est choisi parmi les esters d'acide gras et de monoalcool. De préférence, l'un au moins des alcools et/ou acides est ramifié. On peut citer le myristate d'isopropyle, le palmitate d'isopropyle, l'isononanoate d'isononyle, le palmitate de 2-éthylhexyle, le laurate de 2-hexyldécyle, le palmitate de 2-octyldécyle, le myristate de 2-octyldodécyle, et leurs mélanges.

De préférence, les esters gras sont liquides (à 25°C, 1 atm).

3/ Les alcools gras peuvent être choisis parmi les alcools gras solides à température ambiante et à pression atmosphérique (25°C, 1 atm) et les alcools gras liquides (à 25°C, 1 atm), ainsi que leurs mélanges.

On entend par alcool gras, un alcool aliphatique à longue chaine comprenant de 8 à 40 atomes de carbone, et comprenant au moins un groupe hydroxyle OH. Ces alcools gras ne sont ni oxyalkylénés, ni glycérolés.
De préférence, les alcools gras solides sont de structure R-OH avec R désignant un groupe alkyle linéaire, éventuellement substitué par un ou plusieurs groupes hydroxy, comprenant de 8 à 40, mieux de 10 à 30, voire de 12 à 24 atomes, encore mieux de 14 à 22 atomes de carbone.
Les alcools gras solides susceptibles d'être utilisés peuvent être choisis parmi, seul ou en mélange :
- alcool laurique ou laurylique (1-dodécanol) ;
- alcool myristique ou myristylique (1-tétradécanol) ;
- alcool cétylique (1-hexadécanol) ;
- alcool stéarylique (1-octadécanol) ;
- alcool arachidylique (1-eicosanol) ;
- alcool béhénylique (1-docosanol) ;
- alcool lignocérylique (1-tetracosanol) ;
- alcool cérylique (1-hexacosanol) ;
- alcool montanylique (1-octacosanol) ;
- alcool myricylique (1-triacontanol).

Préférentiellement, l'alcool gras solide est choisi parmi l'alcool cétylique, l'alcool stéarylique, l'alcool béhénylique et leurs mélanges tels que l'alcool cétylstéarylique ou cétéarylique.
Les alcools gras solides peuvent être des mélanges, ce qui signifie que dans un produit commercial peuvent coexister plusieurs espèces notamment de longueur de chaîne différente, sous forme d'un mélange.

Les alcools gras liquides, en particulier ceux en C₁₀-C₃₄ présentent de préférence des chaînes carbonées ramifiées ou possèdent une ou plusieurs, de préférence 1 à 3, insaturations. Ils sont de préférence ramifiés et/ou insaturés, et comportent de 12 à 40 atomes de carbone; et sont non oxyalkylénés et non glycérolés.
Ils présentent de préférence la structure R-OH dans laquelle R désigne de préférence un groupement alkyle ramifié en C12-C24 ou alkényle en C12-C24, R pouvant être substitué par un ou plusieurs groupements hydroxy. De préférence l'alcool gras liquide de l'invention est un alcool saturé ramifié. De préférence, R ne contient pas de groupement hydroxy. On peut notamment citer l'alcool oléique, l'alcool linoléique, l'alcool linolénique, l'alcool isocétylique, l'alcool isostéarylique, le 2-octyl-1-dodécanol, le 2-butyl-octanol, le 2-hexyl-1-décanol, le 2-decyl-1-tétradécanol, le 2-tétradécyl-1-cétanol et leurs mélanges.
Préférentiellement, l'alcool gras liquide est le 2-octyl-1-dodécanol.

De préférence, les alcools gras sont liquides.
4/ Les huiles végétales sont de préférence choisies parmi les triglycérides d'acide gras en C8-C30 et préférentiellement parmi l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de sésame, l'huile de colza, l'huile de carthame, l'huile de coprah, l'huile de maïs, l'huile de noisette, le beurre de karité, l'huile de palme, l'huile de noyau d'abricot, l'huile de calophyllum, les huiles de poisson, le tricaprocaprylate de glycérol.
   On peut également citer les huiles végétales de formule R₉COOR₁₀ dans laquelle R₉ CO représente un radical acyle comportant de 8 à 30 atomes de carbone et R₁₀ représente une chaîne hydrocarbonée linéaire ou ramifiée contenant de 3 à 30 atomes de carbone en particulier alkyle ou alkényle, par exemple, l'huile de Purcellin ou la cire liquide de jojoba.
5/ Les hydrocarbures liquides en C6-C16 peuvent être linéaires ou ramifiés, éventuellement cycliques, et sont de préférence des alcanes. A titre d'exemple, on peut citer l'hexane, le cyclohexane, l'undécane, le dodécane, l'isododécane, le tridécane, le perfluorohexane; et les isoparaffines comme l'isohexadécane, l'isodécane.
   Les hydrocarbures ayant plus de 16 atomes de carbone peuvent être linéaires ou ramifiés, d'origine minérale ou synthétique, et sont choisis de préférence parmi les huiles de paraffine, la vaseline, l'huile de vaseline, les polydécènes, et le polyisobutène hydrogéné tel que Parléam®.
6/ Selon la présente invention, on entend, par céramide, les céramides et/ou les glycocéramides et/ou les pseudocéramides et/ou les néocéramides, naturels ou synthétiques.

Des céramides sont par exemple décrits dans les demandes de brevet DE4424530, DE4424533, DE4402929, DE4420736, WO95/23807, WO94/07844, EP-A-0646572, WO95/16665, FR-2 673 179, EP-A-0227994 et WO 94/07844, WO94/24097, WO94/10131. On préfère les céramides et/ou glycocéramides dont la structure est décrite par DOWNING dans Journal of Lipid Research Vol. 35, 2060-2068, 1994, ou ceux décrits dans la demande de brevet français FR-2 673 179.

Les céramide utilisables selon la présente invention répondent préférentiellement à la formule générale : 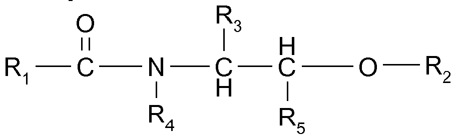 dans laquelle :
- R1 désigne :
   - soit un radical hydrocarboné, linéaire ou ramifié, saturé ou insaturé, en C1-C50, de préférence en C5-C50, ce radical pouvant être substitué par un ou plusieurs groupements hydroxyle éventuellement estérifié par un acide R7COOH, R7 étant un radical hydrocarboné, saturé ou insaturé, linéaire ou ramifié, éventuellement mono ou polyhydroxylé, en C1-C35 , le ou les hydroxyles du radical R7 pouvant être estérifié par un acide gras saturé ou insaturé, linéaire ou ramifié, éventuellement mono ou polyhydroxylé, en C1-C35;
   - soit un radical R"-(NR-CO)-R', où R désigne un atome d'hydrogène ou un radical hydrocarboné C1-C20 mono ou polyhydroxylé, préférentiellement monohydroxylé, R' et R" sont des radicaux hydrocarbonés dont la somme des atomes de carbone est comprise entre 9 et 30, R' étant un radical divalent.
   - soit un radical R8-O-CO-(CH2)p, où R8 désigne un radical hydrocarboné en C1-C20, p est un entier variant de 1 à 12.
- R2 est choisi parmi un atome d'hydrogène, un radical de type saccharidique, en particulier un radical (glycosyle)n, (galactosyle)m ou sulfogalactosyle, un résidu de sulfate ou de phosphate, un radical phosphoryléthylamine et un radical phosphoryléthylammonium, dans lesquels n est un entier variant de 1 à 4 et m est un entier variant de 1 à 8 ;
- R3 désigne un atome d'hydrogène ou un radical hydrocarboné en C1-C33, saturé ou insaturé, hydroxylé ou non, le ou les hydroxyles pouvant être estérifiés par un acide minéral ou un acide R7COOH, R7 ayant les mêmes significations que ci-dessus, le ou les hydroxyles pouvant être éthérifiés par un radical (glycosyle)n, (galactosyle)m, sulfogalactosyle, phosphoryléthylamine ou phosphoryléthylammonium, R3 pouvant également être substitué par un ou plusieurs radicaux alkyle en C1-C14 ;
   de préférence, R3 désigne un radical α-hydroxyalkyle en C15-C26, le groupement hydroxyle étant éventuellement estérifié par un α-hydroxyacide en C16-C30 ;
- R4 désigne un atome d'hydrogène, un radical méthyle, éthyle, un radical hydrocarboné en C3-C50, saturé ou insaturé, linéaire ou ramifié, éventuellement hydroxylé ou un radical -CH2-CHOH-CH2-O-R6 dans lequel R6 désigne un radical hydrocarboné en C10-C26 ou un radical R8-O-CO-(CH2)p, R8 désigne un radical hydrocarboné en C1-C20, p est un entier variant de 1 à 12,
- R₅ désigne un atome d'hydrogène ou un radical hydrocarboné en C1-C30 saturé ou insaturé, linéaire ou ramifié, éventuellement mono ou polyhydroxylé, le ou les hydroxyles pouvant être éthérifiés par un radical (glycosyle)n, (galactosyle)m, sulfogalactosyle, phosphoryléthylamine ou phosphoryléthylammonium,
   sous réserve que lorsque R3=R5=H, ou lorsque R3=H et R5=méthyle, alors R4 ne désigne pas un atome d'hydrogène, un radical méthyle ou un radical éthyle.

Les céramides plus particulièrement préférés sont les composés de formule ci-dessus pour lesquels R1 désigne un alkyle saturé ou insaturé dérivé d'acides gras en C14-C22 éventuellement hydroxylé; R2 désigne un atome d'hydrogène; et R3 désigne un radical linéaire en C11-17 éventuellement hydroxylé et de préférence en C13-17. Encore plus préférentiellement, R3 désigne un radical alpha hydroxycétyle et R2=R4=R5=H.

On peut notamment citer par exemple :
- le 2-N-linoléoylamino-octadécane-1,3-diol,
- le 2-N-oléoylamino-octadécane-1,3-diol (N-oléoyldihydrosphingosine),
- le 2-N-palmitoylamino-octadécane-1,3-diol,
- le 2-N-stéaroylamino-octadécane-1,3-diol,
- le 2-N-béhénoylamino-octadécane-1,3-diol,
- le 2-N-[2-hydroxy-palmitoyl]-amino-octadécane-1,3-diol,
- le 2-N-stéaroyl amino-octadécane-1,3,4 triol et en particulier la N-stéaroyl phytosphingosine,
- le 2-N-palmitoylamino-hexadécane-1,3-diol
ou les mélanges de ces composés.
On peut aussi citer des mélanges spécifiques tels que par exemple les mélanges de céramide(s) 2 et de céramide(s) 5 selon la classification de DOWNING.
On peut également citer les composés de formule ci-dessus pour lesquels R1 désigne un radical alkyle saturé ou insaturé, dérivé d'acides gras en C12-C22; R2 désigne un radical galactosyle ou sulfogalactosyle; et R3 désigne un radical hydrocarboné en C12-C22, saturé ou insaturé, de préférence un ment -CH=CH-(CH₂)₁₂-CH₃.
A titre d'exemple, on peut citer le produit constitué d'un mélange de glycocéramides, vendu sous la dénomination commerciale GLYCOCER par la société WAITAKI INTERNATIONAL BIOSCIENCES.
On peut également utiliser les céramides décrits dans les demandes de brevet EP-A-0227994, EP-A-0 647 617, EP-A-0 736 522 et WO 94/07844.
De tels composés sont par exemple le QUESTAMIDE H (bis-(N-hydroxyéthyl N-cétyl) malonamide) vendu par la société QUEST, le N-(2-hydroxyéthyl)-N-(3-cétyloxy-2-hydroxypropyl)amide d'acide cétylique .
On peut également utiliser le N-docosanoyl N-méthyl-D-glucamine, tel que celui décrit dans la demande de brevet WO94/24097.

7) Les cires végétales ou animales susceptibles d'être utilisées peuvent être choisies notamment parmi la cire de Carnauba, la cire de Candellila, la cire d'olivier, la cire de riz, la cire de jojoba hydrogénée ou les cires absolues de fleurs telles que la cire essentielle de fleur de cassis notamment celle vendue par la société BERTIN (France), les cires d'abeilles, et les cires d'abeilles modifiées (cerabellina).

Préférentiellement, la composition selon l'invention peut comprendre un ou plusieurs agents conditionneurs non polymériques choisis parmi les huiles végétales (triglycérides), les esters gras de préférence liquides, les tensioactifs cationiques, les hydrocarbures liquides en C6-C16, les hydrocarbures ayant plus de 16 atomes de carbone; ainsi que leurs mélanges.

Lesdits agents conditionneurs non polymériques peuvent être présents dans la composition selon l'invention en une quantité allant de 0,1 à 20% en poids, préférentiellement de 0,2 à 10% en poids, mieux encore de 0,2 à 8% en poids, voire de 0,4 à 5% en poids, par rapport au poids total de la composition.

### Particules solides

La composition selon l'invention peut en outre comprendre au moins un type de particules solides; elle peut bien évidemment comprendre plusieurs types différents de particules solides.

Lesdites particules solides sont généralement insolubles dans l'eau.
Par composé insoluble dans l'eau, on entend au sens de la présente invention un composé dont la solubilité dans l'eau à 25°C et à pression atmosphérique est inférieure à 0,1%, mieux inférieure à 0,001%.

Avantageusement, lesdites particules présentent une taille primaire moyenne en nombre allant de 0,001 à 1000 µm, de préférence de 0,01 à 700 µm, préférentiellement de 0,5 à 200 µm.
Au sens de la présente invention, on entend par "taille primaire de particule", la dimension maximale qu'il est possible de mesurer entre deux points diamétralement opposés d'une particule individuelle. La taille des particules peut être déterminée par microscopie électronique à transmission ou à partir de la mesure de la surface spécifique par la méthode BET ou à partir d'une granulométrie laser.

Les particules utilisées dans la composition selon l'invention peuvent présenter différentes formes, par exemple une forme de sphères, de paillettes, d'aiguilles ou de plaquettes; de préférence elles sont sensiblement sphériques.

Lesdites particules peuvent être pleines, creuses ou poreuses.
Lorsque les particules sont creuses, elles comportent généralement au moins une enveloppe continue (ou une couche superficielle) et au moins une cavité. L'enveloppe des particules est de préférence flexible pour se prêter à une déformation mécanique.
Au sens de la présente invention, on entend par "particules poreuses", des particules ayant une structure comportant des pores en nombre et taille variables. La porosité associée à la taille des particules peut être caractérisée quantitativement à partir de la mesure de la surface spécifique par la méthode BET. De préférence, les particules poreuses présentent une surface spécifique supérieure ou égale à 1 m²/g, de préférence supérieure ou égale à 2 m²/g, et préférentiellement supérieure ou égale à 4 m²/g. La surface spécifique est déterminée selon la méthode BET (BRUNAUER-EMMET-TELLER) décrite dans "The journal of the American Chemical Society", vol.60, page 309, février 1938 et correspond à la norme internationale ISO 5794/1 (annexe D). La surface spécifique déterminée par la méthode BET correspond à la surface spécifique totale, micropores compris, des particules considérées.

La composition selon l'invention comprend de préférence des particules d'un ou plusieurs composés organiques, qui peuvent être naturels ou synthétiques, généralement polymériques et/ou des particules d'un ou plusieurs composés minéraux.

Les particules de composés organiques naturels peuvent être notamment des noyaux de fruits micronisés.

Les particules de composés organiques synthétiques non polymériques peuvent être notamment choisies parmi les sels de pyridinethione, notamment les sels de calcium, de magnésium, de baryum, de strontium, de zinc, de cadmium, d'étain et de zirconium. Le sel de zinc de pyridinethione est particulièrement préféré. Un sel de zinc de pyridinethione est notamment commercialisé sous la dénomination Omadine de zinc par la société Arch Personal Care.

Les particules de composés organiques polymériques peuvent être des particules de polymères réticulés ou non.
Ces polymères sont de préférence à l'état vitreux, c'est-à-dire qu'ils présentent une température de transition vitreuse significativement plus élevée que la température ambiante ou la température d'utilisation (par exemple la température du corps humain).
Ainsi que mentionné ci-dessus, lorsque les particules de composés organiques sont creuses, elles comportent au moins une enveloppe continue (ou une couche superficielle) et au moins une cavité. L'enveloppe des particules est de préférence flexible pour se prêter à une déformation mécanique. Elle comprend généralement au moins un polymère, homo- ou copolymère, formé à partir de monomères à insaturation éthylénique.
Les monomères utilisés peuvent être en particulier des esters d'acide méthacrylique ou acrylique, tels que l'acrylate et le méthacrylate de méthyle, le chlorure de vinylidène, l'acrylonitrile, le styrène et ses dérivés.
De préférence, les particules creuses sont des particules creuses déformables d'un copolymère expansé de chlorure de vinylidène et d'acrylonitrile, ou de chlorure de vinylidène, d'acrylonitrile et de (méth)acrylate d'alkyle ou de monomère styrénique. On peut par exemple utiliser un polymère contenant 0-60% de motifs dérivés du chlorure de vinylidène, 20-90% de motifs dérivés d'acrylonitrile et 0-50% de motifs dérivés d'un monomère (méth)acrylique ou styrénique, la somme des pourcentages (en poids) étant égale à 100%. Le monomère (méth)acrylique peut être le (méth)acrylate de méthyle ou d'éthyle. Le monomère styrénique peut être le styrène ou le α-méthyl-styrène.
Préférentiellement, les particules creuses utilisées dans la présente invention sont des particules creuses d'un copolymère expansé de chlorure de vinylidène et d'acrylonitrile ou de chlorure de vinylidène, d'acrylonitrile et de méthacrylate de méthyle. Ces particules peuvent être sèches ou hydratées.
Les particules creuses utilisables dans l'invention sont par exemple les microsphères de terpolymère expansé de chlorure de vinylidène, d'acrylonitrile et de méthacrylate de méthyle, vendues sous la marque EXPANCEL par la société Nobel Casco et en particulier sous les références 551 DE 12 (granulométrie D(0,5) d'environ 12 µm et masse volumique d'environ 40 kg/m³), 551 DE 20 (granulométrie D(0,5) d'environ 15 à 25 µm et masse volumique d'environ 60 kg/m³), 551 DE 50 (granulométrie D(0,5) d'environ 40 µm), 461 DE 50 et 642 WE 50 de 50 µm de granulométrie D(0,5) environ, 551 DE 80 (granulométrie D(0,5) de 50 à 80 µm environ). On peut aussi utiliser des particules de ce même terpolymère expansé, ayant une granulométrie D(0,5) d'environ 18 µm et une masse volumique d'environ 60 à 80 kg/m³ (Expancel EL23) ou encore de granulométrie D(0,5) d'environ 34 µm et de masse volumique d'environ 20 kg/m³. On peut encore citer les particules EXPANCEL 551 DE 40 d42 (granulométrie D(0,5) d'environ 30 à 50 µm et masse volumique d'environ 42 kg/m³), 551 DE 80 d42 (granulométrie D(0,5) d'environ 50 à 80 µm et masse volumique d'environ 42 kg/m³), 461 DE 20 d70 (granulométrie D(0,5) d'environ 15 à 25 µm et masse volumique d'environ 70 kg/m³), 461 DE 40 d25 (granulométrie D(0,5) d'environ 35 à 55 µm et masse volumique d'environ 25 kg/m³), 461 DE 40 d60 (granulométrie D(0,5) d'environ 20 à 40 µm et masse volumique d'environ 60 kg/m³), 461 DET 40 d25 (granulométrie D(0,5) d'environ 35 à 55 µm et masse volumique d'environ 25 kg/m³), 051 DE 40 d60 (granulométrie D(0,5) d'environ 20 à 40 µm et masse volumique d'environ 60 kg/m³), 091 DE 40 d30 (granulométrie D(0,5) d'environ 35 à 55 µm et masse volumique d'environ 30 kg/m³), 091 DE 80 d30 (granulométrie D(0,5) d'environ 60 à 90 µm et masse volumique d'environ 30 kg/m³). On peut encore utiliser des particules de polymère de chlorure de vinylidène et d'acrylonitrile ou de chlorure de vinylidène, d'acrylonitrile et de méthacrylate de méthyle non expansé comme celles vendues sous la marque EXPANCEL avec la référence 551 DU 10 (granulométrie D(0,5) d'environ 10 µm) ou 461 DU 15 (granulométrie D(0,5) d'environ 15 µm).

Les particules d'un ou plusieurs composés organiques susceptibles d'être utilisées dans la composition cosmétique selon l'invention peuvent être notamment choisies parmi les poudres de polyamide; les poudres de polymères acryliques, notamment de polyacrylate de sodium réticulé, de polyméthacrylate de méthyle; les poudres de copolymères acryliques, notamment de polyméthacrylate de méthyle/diméthacrylate d'éthylène glycol, de polyméthacrylate d'allyle/diméthacrylate d'éthylène glycol, de copolymère diméthacrylate d'éthylène glycol/méthacrylate de lauryle, de polyacrylate/alkylacrylate; les poudres de polystyrène; les poudres de polyéthylène, notamment de polyéthylène/acide acrylique; les microbilles de résine de silicone.
On peut plus particulièrement citer en tant que particules de composés organiques selon l'invention :
- les poudres de polyamides (Nylon ®), par exemples celles commercialisées sous les dénominations « ORGASOL ® 4000 » et « ORGASOL ® 2002 UD NAT COS 204 » par la société ATOCHEM,
- les poudres de polymères acryliques, notamment de polyacrylate de sodium réticulé, comme par exemple celles commercialisées sous la dénomination ASAP 2000 par la société CHEMOAL ou HYSORB M7055 pour la société BASF, de polyméthacrylate de méthyle, comme par exemples celles commercialisées sous la dénomination « COVABEAD ® LH85 » « COVABEAD ® PMMA » par la société SENSIENT ou celles commercialisées sous la dénomination « MICROPEARL ® MHB », « MICROPEARL® M 100 », « MICROPEARL® M 310» commercialisée par la société MATSUMOTO,
- les poudres de copolymères acryliques, notamment de polyméthacrylate de méthyle/diméthacrylate d'éthylène glycol, comme celles commercialisées sous la dénomination de « DOW CORNING 5640 MICROSPONGE ® SKIN OIL ADSORBER » par la société DOW CORNING, ou celles commercialisés sous la dénomination « GANZPEARL ® GMP-0800 » par la société AICA KOGYO, de polyméthacrylate d'allyle/diméthacrylate d'éthylène glycol, comme celles commercialisées sous la dénomination « POLYPORE ® L200 » ou « POLYPORE ® E200 » commercialisés par la société AMCOL, de copolymère diméthacrylate d'éthylène glycol / méthacrylate de lauryle, comme celles commercialisées par « POLYTRAP ® 6603 » par la société DOW CORNING, de polyacrylate/éthylhexylacrylate comme celles commercialisées sous la dénomination « TECHPOLYMER ® ACX 806C » par la société SEKISUI,
- les poudres de polystyrène telles que celles commercialisées sous la dénomination "POLYSPHERE 3 000 SP" par la Société PRESPERESE,
- les poudres de polystyrène / dinvinylbenzène comme celles commercialisées sous la dénomination « TECHPOLYMER ® SBX8 » par la société SEKISUI,
- les poudres de copolymère styrène/acrylate comme celles commercialisées sous la dénomination « SUNSPHERES POWDER » par la société ROHM & HAAS,
- les poudres de polyéthylène, notamment de polyéthylène/acide acrylique commercialisées sous la dénomination « FLOBEADS ® » par la société SUMITOMO, ou les billes de polyéthylène commercialisées sous la dénomination « MICROPOLY 220 L » par la société MICRO POWDERS,
- les microbilles de résine de silicone, comme celles commercialisées sous les dénominations « TOSPEARL ® » par la société TOSHIBA SILICONE, en particulier les « TOSPEARL ® 240A » et « TOSPEARL ® 120A ».

De préférence, les particules de composés organiques (ou particules organiques) sont choisies parmi les poudres de polyamide, les poudres de polyacrylate de sodium réticulé, les poudres de polyéthylène et les poudres de polyméthylméthacrylate.

Lesdites particules organiques peuvent être éventuellement traitées en surface par un agent de traitement hydrophobe. Ainsi les particules organiques peuvent être rendues hydrophobes par enrobage ou greffage chimique par des produits tels que : les silicones, comme les méthicones ou les diméthicones; les acides aminés, les acides aminés N-acylés ou leurs sels; les savons métalliques comme le dimyristate d'aluminium, le sel d'aluminium du glutamate de suif hydrogéné; les dérivés fluorés, comme par exemple les perfluoroalkylphosphates, les perfluoroalkylsilanes, les polyoxydes d'hexafluoropropylène, les polyorganosiloxanes comprenant des groupes perfluroalkyl perfluoropolyéthers; la lécithine, le triisostéaroyl titanate d'isopropyle; les acides gras comme l'acide stéarique.
Le terme alkyle mentionné dans les composés cités ci-dessus peut désigner notamment un groupe alkyle, linéaire, ramifié ou cyclique comprenant de 1 à 30 atomes de carbone, notamment de 5 à 16 atomes de carbone.
Les acides aminés N-acylés peuvent comprendre un groupe acyle comprenant de 8 à 22 atomes de carbone, comme par exemple un groupe 2-éthyl hexanoyle, caproyle, lauroyle, myristoyle, palmitoyle, stéaroyle et cocoyle.
Les sels de ces composés peuvent être les sels d'aluminium, de magnésium, de calcium, de zirconium, de zinc de sodium ou de potassium.
L'acide aminé peut être par exemple la lysine, l'acide glutamique ou l'alanine.
A titre d'exemple, on peut citer les microsphères de poly méthacrylate de méthyle enrobées de tri-isostéaryl titanate d'isopropyle de taille 2-15 µm commercialisées par Kobo sous la référence BPA-515.

Les particules d'un ou plusieurs composés minéraux (ou particules minérales) susceptibles d'être utilisées dans la composition cosmétique selon l'invention peuvent être choisies parmi les particules métalliques, les oxydes, les sels inorganiques, les carbures, les nitrures, les borures, les sulfures et les hydroxydes.
Par particules métalliques, on entend des particules formées par des métaux, notamment choisis parmi les métaux alcalino-terreux, les métaux de transition, les métaux des terres rares et les alliages de ces métaux.
De préférence, les métaux utilisés sont le bore, l'aluminium, le cuivre, le cadmium, le sélénium, l'argent, l'or, l'indium, le fer, le platine, le nickel, le molybdène, le silicium, le titane, le tungstène, l'antimoine, le palladium, le zinc, l'étain et les alliages de ces métaux. Parmi ces métaux, on préfère tout particulièrement l'or, l'argent, le platine, le cadmium, le sélénium et les alliages de ces métaux.
Parmi ces composés, on peut en particulier citer le disulfure de sélénium.

Les particules d'un ou plusieurs composés minéraux peuvent être également des oxydes. On peut citer les oxydes des éléments des colonnes 1 à 14 du tableau de classification périodique des éléments. En particulier, on peut notamment citer les oxydes de titane, de zinc, de cérium, de zirconium, d'aluminium et d'oxychlorure de bismuth. Parmi ces composés, on préfère tout particulièrement l'oxyde de zinc. Les particules d'un ou plusieurs composés minéraux peuvent être des sels inorganiques. On peut notamment citer le sulfate de baryum, le carbonate de calcium, le sulfate de calcium, le phosphate de calcium, l'hydrocarbonate de magnésium. Parmi ces composés, on préfère le carbonate de calcium.

Parmi les particules d'un ou plusieurs composés minéraux appartenant aux espèces décrites ci-dessus, on peut aussi citer les argiles, les silicates, l'alumine, la silice, le kaolin et l'hydroxyapatite.
En particulier, les silices utilisables peuvent être naturelles et non traitées. On peut ainsi citer les silices proposées sous les dénominations SILLITIN N85, SILLITIN N87, SILLITIN N82, SILLITIN V85 et SILLITIN V88 par la société HOFFMANN MINERAL, ou SUNSIL 130 par la société SUNJIN CHEMICAL, MSS-500-3 H par la société Kobo, SUNSPHERE H 51 par la société AGC SI-TECH, les particules creuses de silice amorphe de forme ellipsoïdale commercialisées par Kobo sous la référence silica shells. Elles peuvent être pyrogénées. Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leur surface.
Il est possible de modifier chimiquement la surface de ladite silice, par réaction chimique générant une diminution du nombre de groupes silanol. On peut notamment substituer des groupes silanol par des groupements hydrophobes; on obtient alors une silice hydrophobe. Les groupements hydrophobes peuvent être :
(a) des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995).
(b) des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénommées "Silica diméthyl silylate" selon le CTFA (6ème édition, 1995).
En particulier, parmi les silices hydrophobes, on peut citer les aérogels de silice. Les aérogels sont des matériaux poreux ultra légers, dont les premiers ont été réalisés par Kristler en 1932. Ils sont généralement synthétisés par un procédé sol-gel en milieu liquide puis séchés par extraction d'un fluide supercritique. Le fluide supercritique le plus communément utilisé est le CO2 supercritique. Ce type de séchage permet d'éviter la contraction des pores et du matériau. D'autres types de séchage permettent également d'obtenir des matériaux poreux à partir de gel, à savoir (i) le séchage par cryodessiccation, consistant à solidifier à faible température le gel puis à sublimer le solvant et (ii) le séchage par évaporation. Les matériaux ainsi obtenus sont appelés respectivement cryogels et xérogels. Le procédé sol-gel et les différents séchages sont décrits en détail dans Brinker CJ., and Scherer G.W., Sol-Gel Science: New York: Academic Press, 1990.
Par « silice hydrophobe », on entend toute silice dont la surface est traitée par des agents de silylation, par exemple par des silanes halogénés tels que des alkylchlorosilanes, des siloxanes, en particulier des dimethylsiloxanes tel que l'hexamethyldisiloxane, ou des silazanes, de manière à fonctionnaliser les groupements OH par des groupements silyles Si-Rn, par exemple des groupements triméthylsilyles.
De préférence, les particules d'aérogel hydrophobe pouvant être utilisées dans la présente invention présentent avantageusement une surface spécifique par unité de masse (SM) allant de 500 à 1500 m2/g, de préférence de 600 à 1200 m2/g et mieux de 600 à 800 m2/g et/ou ont une capacité d'absorption d'huile mesurée au WET POINT allant de 5 à 18 ml/g de particules, de préférence de 6 à 15 ml/g et mieux de 8 à 12 ml/g.
La capacité d'absorption mesurée au Wet Point, et notée Wp, correspond à la quantité d'huile qu'il faut additionner à 100 g de particules pour obtenir une pâte homogène. Elle est mesurée selon la méthode dite de Wet Point ou méthode de détermination de prise d'huile de poudre selon le principe décrit dans la norme NF T 30-022. Elle correspond à la quantité d'huile adsorbée sur la surface disponible de la poudre et/ou absorbée par la poudre par mesure du Wet Point, décrite ci-dessous : on place une quantité m= 2 g de poudre sur une plaque de verre puis on ajoute goutte à goutte l'huile (isononyl isononanoate). Après addition de 4 à 5 gouttes d'huile dans la poudre, on mélange à l'aide d'une spatule et on continue d'ajouter de l'huile jusqu'à la formation de conglomérats d'huile et de poudre. A partir de ce moment, on ajoute l'huile à raison d'une goutte à la fois et on triture ensuite le mélange avec la spatule. On cesse l'addition d'huile lorsque l'on obtient une pâte ferme et lisse. Cette pâte doit se laisser étendre sur la plaque de verre sans craquelures ni formation de grumeaux. On note alors le volume Vs (exprimé en ml) d'huile utilisé. La prise d'huile correspond au rapport Vs / m.
Les particules d'aérogel de silice hydrophobe utilisées selon la présente invention sont de préférence des particules d'aérogel de silice silylée (nom INCI silica silylate).
La préparation de particules d'aérogels de silice hydrophobe modifiées en surface par silylation est décrite plus avant dans le document US 7,470,725. On utilisera en particulier des particules d'aérogels de silice hydrophobe modifiée en surface par groupements triméthylsilyles.
Les particules d'aérogel hydrophobe pouvant être utilisées dans la présente invention présentent avantageusement une taille, exprimée en diamètre moyen (D[0,5]), inférieure à 1500 µm et de préférence allant de 1 à 30 µm, de préférence de 5 à 25 µm, mieux de 5 à 20 µm et encore mieux de mieux de 5 à 15 µm..
Les tailles des particules d'aérogel selon l'invention peuvent être mesurées par diffusion statique de la lumière au moyen d'un granulomètre commercial de type MasterSizer 2000 de chez Malvern. Les données sont traitées sur la base de la théorie de diffusion de Mie. Cette théorie, exacte pour des particules isotropes, permet de déterminer dans le cas de particules non sphériques, un diamètre « effectif » de particules. Cette théorie est notamment décrite dans l'ouvrage de Van de Hulst, H.C., "Light Scattering by Small Particles," Chapitres 9 et 10, Wiley, New York, 1957.
Selon un mode de réalisation avantageux, les particules d'aérogel hydrophobe utilisées dans la présente invention présentent une surface spécifique par unité de masse (SM) allant de 600 à 800 m2/g et une taille exprimée en diamètre moyen en volume (D[0,5]) allant de 5 à 20 µm, mieux de 5 à 15 µm.
Selon un mode de réalisation préféré, on utilisera plus particulièrement le VM-2270, dont les particules présentent une taille moyenne allant de 5 à 15 microns et une surface spécifique par unité de masse allant de 600 à 800 m2/g.
Les particules d'aérogel hydrophobe utilisées dans la présente invention peuvent avantageusement présenter une densité tassée p allant de 0,04 g/cm3 à 0,10 g/cm3, de préférence de 0,05 g/cm3 à 0,08 g/cm3.
Dans le cadre de la présente invention, cette densité peut être appréciée selon le protocole suivant, dit protocole de la densité tassée : 40 g de poudre sont versés dans une éprouvette graduée puis l'éprouvette est placée sur un appareil STAV 2003 de chez STAMPF VOLUMETER. L'éprouvette est ensuite soumise à une série de 2500 tassements (cette opération est recommencée jusqu'à ce que la différence de volume entre 2 essais consécutifs soit inférieure à 2 %); puis le volume final Vf de poudre tassée est mesuré directement sur l'éprouvette. La densité tassée est déterminée par le rapport masse(m)/Vf, en l'occurrence 40/Vf (Vf étant exprimé en cm3 et m en g).
Selon un mode de réalisation, les particules d'aérogel hydrophobes utilisées dans la présente invention présentent une surface spécifique par unité de volume SV allant de 5 à 60 m2/cm3, de préférence de 10 à 50 m2/cm3 et mieux de 15 à 40 m2/cm3.
La surface spécifique par unité de volume est donnée par la relation : SV = SM*p où p est la densité tassée exprimée en g/cm3 et SM est la surface spécifique par unité de masse exprimée en m2/g, telles que définies plus haut.
Selon un mode de réalisation préféré, les particules d'aérogel hydrophobe selon l'invention présentent une surface spécifique par unité de masse (SM) allant de 500 à 1500 m2/g, de préférence de 600 à 1200 m2/g et mieux de 600 à 800 m2/g, et une taille exprimée en diamètre moyen (D[0,5]) allant de 1 à 30 µm et/ou une capacité d'absorption d'huile mesurée au WET POINT allant de 5 à 18 ml/g de particules, de préférence de 6 à 15 ml/g et mieux de 8 à 12 ml/g.
A titre d'aérogels de silice hydrophobe utilisables dans l'invention, on peut citer par exemple l'aérogel commercialisé sous la dénomination VM-2260 (nom INCI Silica silylate), par la société Dow Corning, dont les particules présentent une taille moyenne d'environ 1000 microns et une surface spécifique par unité de masse allant de 600 à 800 m2/g.
On peut également citer les aérogels commercialisés par la société Cabot sous les références AEROGEL TLD 201, AEROGEL OGD 201 et AEROGEL TLD 203, ENOVAAEROGEL MT 1100, ENOVAAEROGEL MT 1200.
On utilisera plus particulièrement l'aérogel commercialisé sous la dénomination VM-2270 (nom INCI Silica silylate), par la société Dow Corning, dont les particules présentent une taille moyenne allant de 5 à 15 microns et une surface spécifique par unité de masse allant de 600 à 800 m2/g.

Les particules minérales selon l'invention peuvent être aussi des particules d'argile. Les argiles sont des produits déjà bien connus en soi, qui sont décrits par exemple dans l'ouvrage "Minéralogie des argiles, S. Caillère, S. Hénin, M. Rautureau, 2ème édition 1982, Masson".
Les argiles sont des silicates contenant un cation pouvant être choisi parmi les cations de calcium, de magnésium, d'aluminium, de sodium, de potassium, de lithium et leurs mélanges.
On peut notamment citer les argiles de la famille des smectites telles que les montmorillonites, les hectorites, les bentonites, les beidellites, les saponites, ainsi que de la famille des vermiculites, de la stévensite, des chlorites.
Les argiles peuvent être d'origine naturelle ou synthétique. De préférence, on utilise les argiles qui sont cosmétiquement compatibles et acceptables avec les fibres kératiniques telles que les cheveux.
L'argile peut être choisie parmi la montmorillonite, la bentonite, l'hectorite, l'attapulgite, la sépiolite, et leurs mélanges. De préférence, l'argile est une bentonite ou une hectorite.
Les argiles peuvent être choisies parmi les argiles organophiles. Les argiles organophiles sont des argiles modifiées avec un composé chimique choisi parmi les amines quaternaires, les amines tertiaires, les acétates aminés, les imidazolines, les savons aminés, les sulfates gras, les alkyl aryl sulfonates, les oxydes amines, et leurs mélanges. De préférence, les argiles organophiles selon l'invention sont des argiles modifiées avec un composé chimique choisi parmi les amines quaternaires. Comme argiles organophiles, on peut citer les quaternium-18 bentonites telles que celles vendues sous les dénominations Bentone 3, Bentone 38, Bentone 38V par la société Elementis, Tixogel VP par la société United catalyst, Claytone 34, Claytone 40, Claytone XL par la société Southern Clay; les stéaralkonium bentonites telles que celles vendues sous les dénominations Bentone 27V par la société Elementis, Tixogel LG par la société United Catalyst, Claytone AF, Claytone APA par la société Southern Clay et les quaternium-18/benzalkonium bentonite telles que celles vendues sous les dénominations Claytone HT, Claytone PS par la société Southern Clay. L'argile organophile est en particulier choisie parmi les hectorites modifiées telles que l'hectorite modifiée par du chlorure d'ammonium d'acide gras en C10-C12, notamment du chlorure de distéaryle diméthylammonium et du chlorure de stéaryle de benzyldiméthylammonium.
De préférence, les particules d'un ou plusieurs composés minéraux sont choisies parmi le carbonate de calcium, la silice et en particulier les aérogels de silice.

Les particules solides, notamment insolubles dans l'eau, peuvent également être choisies parmi les pigments colorés organiques ou minéraux, ou encore les agents nacrants non colorés.
Les pigments organiques colorés peuvent être choisis parmi la laque de Carmin, le noir d'aniline, le jaune azo, la quinacridone, le bleu de phtalocyanine, les pigments bleus codifiés dans le Color Index sous les références Cl 42090, 69800, 69825, 73000, 74100, 74160, les pigments jaunes codifiés dans le Color Index sous les références Cl 11680, 11710, 15985, 19140, 20040, 21100, 21108, 47000, 47005, les pigments verts codifiés dans le Color Index sous les références Cl 61565, 61570, 74260, les pigments oranges codifiés dans le Color Index sous les références Cl 11725, 15510, 45370, 71105, les pigments rouges codifiés dans le Color Index sous les références Cl 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.
On peut aussi citer les pâtes pigmentaires de pigment organique telles que les produits vendus par la société HOECHST sous le nom :
- JAUNE COSMENYL IOG : Pigment YELLOW 3 (Cl 11710) ;
- JAUNE COSMENYL G : Pigment YELLOW 1 (Cl 11680) ;
- ORANGE COSMENYL GR : Pigment ORANGE 43 (Cl 71105) ;
- ROUGE COSMENYL R : Pigment RED 4 (Cl 12085) ;
- CARMIN COSMENYL FB : Pigment RED 5 (Cl 12490) ;
- VIOLET COSMENYL RL : Pigment VIOLET 23 (Cl 51319) ;
- BLEU COSMENYL A2R : Pigment BLUE 15.1 (Cl 74160) ;
- VERT COSMENYL GG : Pigment GREEN 7 (Cl 74260) ;
- NOIR COSMENYL R : Pigment BLACK 7 (Cl 77266).

On peut encore citer, comme pigments, les pigments nacrés tels les pigments nacrés colorés tels que le mica recouvert de titane et d'oxydes de fer, le mica recouvert d'oxyde de fer, le mica recouvert de titane et notamment de bleu ferrique ou d'oxyde de chrome, le mica recouvert de titane et d'un pigment organique tel que défini précédemment ainsi que les pigments nacrés à base d'oxychlorure de bismuth. Comme pigments nacrés, on peut citer les nacres Cellini commercialisée par Engelhard (Mica-TiO₂-laque), Prestige commercialisée par Eckart (Mica-TiO₂), Prestige Bronze commercialisée par Eckart (Mica-Fe₂O₃), Colorona commercialisée par Merck (Mica-TiO₂-Fe2O₃); les nacres de couleur or notamment commercialisées par la société ENGELHARD sous le nom de Brillant gold 212G (Timica), Gold 222C (Cloisonne), Sparkle gold (Timica), Gold 4504 (Chromalite) et Monarch gold 233X (Cloisonne); les nacres bronzes notamment commercialisées par la société MERCK sous la dénomination Bronze fine (17384) (Colorona) et Bronze (17353) (Colorona) et par la société ENGELHARD sous la dénomination Super bronze (Cloisonne); les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna); les nacres de teinte brune notamment commercialisées par la société ENGELHARD sous la dénomination Nuantique copper 340XB (Cloisonne) et Brown CL4509 (Chromalite); les nacres à reflet cuivre notamment commercialisées par la société ENGELHARD sous la dénomination Copper 340A (Timica); les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite); les nacres de teinte rouge à reflet or notamment commercialisées par la société ENGELHARD sous la dénomination Sunstone G012 (Gemtone); les nacres roses notamment commercialisées par la société ENGELHARD sous la dénomination Tan opale G005 (Gemtone); les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

Parmi les agents nacrants, on peut citer plus particulièrement :
- les esters de polyols ayant au moins deux atomes de carbone et d'acides gras à chaîne longue, préférentiellement en C₁₀-C₃₀ et encore plus préférentiellement en C₁₆-C₂₂ ; tels que des mono ou diesters de polyols et d'acides gras; de préférence les polyols sont l'éthylène glycol et les polyalkylène glycol ayant de 2 à 10 motifs d'oxyde d'éthylène ;
- les éthers d'alcools gras à chaîne longue solides à une température inférieure ou égale à environ 30°C tels que par exemple les dialkyléthers de formule : R-O-R', dans laquelle R et R', identiques ou différents, désignent un radical alkyle, saturé ou insaturé, linéaire ou ramifié, comportant de 10 à 30 atomes de carbone et de préférence de 14 à 24 atomes de carbone, R et R' étant choisis de façon telle que le composé soit solide à une température inférieure ou égale à 30°C environ.
   Plus particulièrement, R et R' désignent un radical stéaryle. Un distéaryléther utilisable dans le cadre de la présente invention, est commercialisé sous la dénomination CUTINA STE par la société HENKEL.
- les cyclodextrines et en particulier la β-cyctodextrine.

Les agents nacrants sont préférentiellement choisis parmi les mono- ou distéarate d'éthylène glycol (ou distéarate de glycol), le distéaryléther, le 1-(hexadécyloxy)-2-octadécanol, la β-cyciodextrine et leurs mélanges.

Préférentiellement, la composition selon l'invention comprend des particules solides choisies parmi les silices, les argiles, les pigments, les agents nacrants, les sels de pyridinethione, le disulfure de sélénium, et tout particulièrement parmi les silices, les agents nacrants tels que les mono- ou distéarate d'éthylène glycol, le distéaryléther, le 1-(hexadécyloxy)-2-octadécanol, la β-cyctodextrine; le sel de zinc de pyridinethione, et le disulfure de sélénium, et leurs mélanges.

Les particules solides sont de préférence présentes dans la composition en une quantité allant de 0,001 à 15% en poids, préférentiellement de 0,1 à 10% en poids, mieux encore de 0,5 à 8% en poids, par rapport au poids total de la composition.

### Agents antipelliculaires

La composition selon l'invention peut en outre comprendre au moins un agent antipelliculaire; elle peut bien évidemment comprendre plusieurs agents antipelliculaires. Cet agent antipelliculaire peut être particulaire ou non particulaire.

Les agents antipelliculaires susceptibles d'être utilisés selon l'invention sont notamment choisis parmi les familles suivantes :
1) les sels de pyridinethione notamment les sels de calcium, de magnésium, de baryum, de strontium, de zinc, de cadmium, d'étain et de zirconium. Le sel de zinc de pyridinethione est particulièrement préféré.
   Le sel de zinc de pyridinethione est notamment commercialisé sous la dénomination Omadine de zinc par la société Arch Personal Care.
2) les dérivés de 1-hydroxy-2-pyrrolidone notamment représentés par la formule : 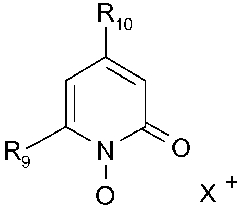 dans laquelle :
   - Rg représente un groupe alkyle ayant de 1 à 17 atomes de carbone, alcényle ayant de 2 à 17 atomes de carbone, un groupe cycloalkyle ayant de 5 à 8 atomes de carbone, un groupe bicycloalkyle ayant de 7 à 9 atomes de carbone ; un groupe cycloalkyl-alkyle, un groupe aryle, un groupe aralkyle avec un alkyle ayant de 1 à 4 atomes de carbone, un groupe arylalcényle avec un alcényle ayant de 2 à 4 atomes de carbone, aryloxyalkyle ou arylmercaptoalkyle avec un alkyle ayant de 1 à 4 atomes de carbone, un groupe furylalcényl avec un alcényle ou un furyle ayant de 2 à 4 atomes de carbone, un groupe alcoxy ayant de 1 à 4 atomes de carbone, un groupe nitro, un groupe cyano ou un atome d'halogène.
   - R₁₀ représente un atome d'hydrogène, un groupe alkyle en C₁-C₄, un groupe alcényle en C₂-C₄, un atome d'halogène, un groupe phényle ou un groupe benzyle ;
   - X représente une base organique ou inorganique, un ion de métal alcalin ou alcalinoterreux, un ion ammonium.

   On peut notamment citer la 1-hydroxy-4-méthyl-2-pyridone, la 1-hydroxy-6-méthyl-2-pyridone, la 1-hydroxy-4,6-diméthyl-2-pyridone, la 1-hydroxy-4-méthyl-6-(2,4,4-triméthylpentyl)-2-pyridone, la 1-hydroxy-4-méthyl-6-cyclohexyl-2-pyridone, la 1-hydroxy-4-méthyl-6-(méthyl-cyclohexyl)-2-pyridone, la 1-hydroxy-4-méthyl-6-(2-bicyclo[2,2,1]heptyl)-2-pyridone, la 1-hydroxy-4-méthyl-6-(4-méthyl-phényl)-2-pyridone, la 1-hydroxy-4-méthyl-6-[1-(4-nitrophénoxy)-butyl]-2-pyridone, la 1-hydroxy-4-méthyl-6-(4-cyanophénoxymethyl)-2-pyridone, la 1-hydroxy-4-méthyl-6-(phénylsulfonylméthyl)-2-pyridone, et la 1-hydroxy-4méthyl-6-(4-bromo-benzyl)-2-pyridone.
   Ces composés peuvent être utilisés sous forme de sels avec des bases organiques ou inorganiques. Des exemples de bases organiques sont notamment les alcanolamines de faibles poids moléculaires telles que l'éthanolamine, la diéthanolamine, la N-éthyléthanolamine, la triéthanolamine, le diéthylaminoéthanol, le 2-amino-2-méthylpropanediol; les bases non volatiles telles que l'éthylènediamine, l'hexaméthylènediamine, la cyclohexylamine, la benzylamine et la N-méthylpipérazine; les hydroxydes d'ammonium quaternaires, comme l'hydroxyde de triméthylbenzylammonium; la guanidine et ses dérivés, et particulièrement ses dérivés alkylés. Des exemples de bases inorganiques sont notamment les sels de métal alcalins, par exemple sodium, potassium; les sels d'ammonium, les sels de métaux alcalino-terreux, comme le magnésium et le calcium; les sels de métaux di, tri ou tétravalents cationiques, tels que zinc, aluminium, zirconium.
   Les alcanolamines, l'éthylènediamine, et les bases inorganiques telles que les sels de métal alcalin sont préférés. Un composé particulièrement préféré est celui pour lequel Rg désigne le roue : 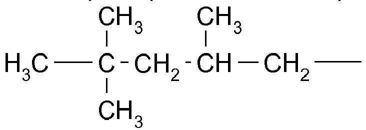 R₁₀ désigne un groupe méthyle et X⁺ désigne N⁺H₃CH₂CH₂OH.
   Ce composé est par exemple commercialisé sous la dénomination Octopirox (1-hydroxy-4-méthyl-6-(2,4,4-triméthylpentyl)-2-pyridone, sel de monoéthanolamine) par la société HOECHST.
3) le 2,2'-dithio-bis-(pyridine-N-oxide) de formule : 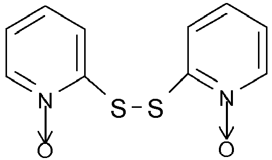
   Ces composés peuvent être introduits dans les compositions sous forme de sels inorganiques. Des exemples de sels inorganiques sont le sulfate de magnésium.
   (VI)
4) les trihalogénocarbamides notamment de formule 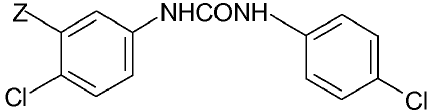 dans laquelle Z représente un atome d'halogène comme le chlore, ou un groupement trihalogénoalkyle en C₁-C₄ tel que CF₃.
5) le triclosan représenté par la formule: 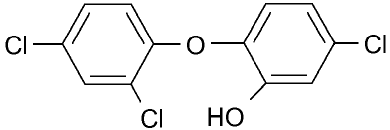
6) les composés azolés tels que le climbazole, le kétoconazole, le clotrinazole, l'éconazole, l'isoconazole et le miconazole.
7) les polymères antifongiques tels que l'amphotéricine B ou la nystatine.
8) les sulfures de sélénium en particulier ceux de formule SₓSe₈₋ₓ, x allant de 1 à 7.
9) les extraits d'une ou plusieurs bactéries filamenteuses non photosynthétiques non fructifiantes.
   Les extraits bactériens utilisables selon l'invention sont notamment choisis parmi les bactéries filamenteuses non photosynthétiques et non fructifiantes telles que définies selon la classification du Bergey's Manual of Systemic Bacteriology, volume 3, section 23, 9ème édition 1989.
   Parmi les bactéries utilisables, on citera plus particulièrement les bactéries appartenant à l'ordre des Beggiatoales, et notamment les bactéries appartenant au genre Beggiotoa, telles que par exemple diverses souches de Beggiotoa alba. Suivant la définition B. alba correspond aux anciennes appellations Beggiotoa arachnoidea, B. gigantea, B. leptomiformis, B. minima, B. mirabilis du Bergey's manual, 8ème édition. On peut citer par ailleurs les bactéries appartenant au genre Vitreoscilla, dont on sait qu'il est proche et souvent difficilement discernable du genre Beggiatoa. Les bactéries qui viennent d'être définies, et dont plusieurs ont été décrites, ont généralement un habitat aquatique, et peuvent être trouvées notamment dans les sources d'eau thermale. Parmi les bactéries utilisables, on peut citer par exemple, Vitreoscilla beggiatoïdes (ATCC 43181) et Beggiatoa alba (ATCC33555); préférentiellement, l'utilisation de l'extrait de Vitreoscilla filiformis, en particulier, la souche ATCC 15551, ses métabolites et ses fractions.
   D'autre part, il est connu que la culture des bactéries filamenteuses non photosynthétiques et non fructifiantes est relativement difficile, de même que l'obtention de cultures pures. On utilisera préférentiellement la culture décrite dans la demande de brevet WO 94/02158.
   Par extrait de bactéries filamenteuses non photosynthétiques et non fructifiantes, on entend aussi bien le surnageant de culture, la biomasse obtenue après culture des dites bactéries, les enveloppes ou fractions d'enveloppes, ou les extraits de la biomasse obtenus par traitement de cette biomasse.
   Pour préparer l'extrait selon l'invention, on peut cultiver lesdites bactéries puis les séparer de la biomasse obtenue par exemple par filtration, centrifugation, coagulation et/ou lyophilisation.
   On peut notamment préparer les extraits utilisables selon le procédé décrit dans la demande de brevet WO-A-93/00741. Ainsi, après culture, les bactéries sont concentrées par centrifugation. La biomasse obtenue est autoclavée. Cette biomasse peut-être lyophilisée pour constituer ce que l'on appelle l'extrait lyophilisé. Toute méthode de lyophilisation connue de l'homme du métier est utilisable pour préparer cet extrait.
   La fraction surnageante de cette biomasse peut également être filtrée dans un récipient stérile pour éliminer les particules en suspension.
   On appelle ici enveloppes ou fractions d'enveloppes, la paroi bactérienne et éventuellement les membranes sous jacentes.
10) l'acide ellagique, ses éthers, les sels de l'acide ellagique et les sels de ses éthers; ainsi que les tannins d'acide ellagique.
   L'acide ellagique, également dénommé 2,3,7,8-tétra-hydroxy(1)-benzopyrano (5,4,3-cde)(1)benzopyran-5,10 dione est une molécule bien connue et présente dans le règne végétal. On pourra se reporter à la publication du Merck Index 20ème édition (1996), n° 3588. L'acide ellagique présente la formule chimique suivante : 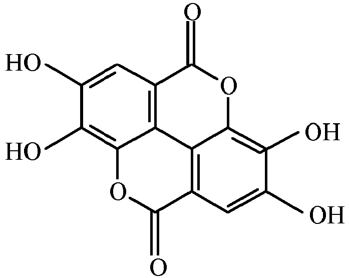 qui comporte quatre cycles accolés.
   On connaît par le document FR-A-1 478 523, un procédé de purification de l'acide ellagique ainsi que les acides ellagiques purifiés obtenus par un tel procédé. Le ou les éthers de l'acide ellagique utilisables selon l'invention, sont de préférence choisis parmi les mono-, di-, tri- ou polyéthers obtenus par éthérification d'un ou plusieurs groupes hydroxyle (un des quatre groupes OH de l'acide ellagique) de l'acide ellagique en un ou plusieurs groupes OR, R étant choisi(s) parmi les groupements alkyle en C₂-C₂₀, les groupements polyoxyalkylènes et en particulier polyoxyéthylènes et/ou polyoxypropylènes, et les groupements dérivés d'un ou plusieurs mono ou polysaccharides tels que par exemple le groupement de formule suivante : 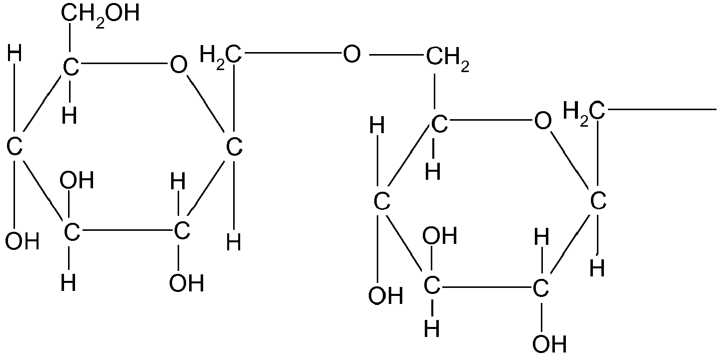
   Dans le cas des di-, tri- ou polyéthers de l'acide ellagique, les groupes R tels que définis ci-dessus peuvent être identiques ou différents.
   De tels éthers sont décrits dans le brevet US 5,073,545. De préférence ces éthers de l'acide ellagique sont choisis parmi l'acide 3,4-di-O-méthyl ellagique, l'acide 3,3',4-tri-O-méthyl ellagique et l'acide 3,3'-di-O-méthyl ellagique.
   Le ou les sels de l'acide ellagique et/ou de ses éthers utilisables selon l'invention, sont de préférence choisis parmi les sels de métaux alcalins ou alcalino-terreux, tels que le sodium, le potassium, le calcium et le magnésium, le sel d'ammonium et les sels d'amines tels que les sels de triéthanolamine, de monoéthanolamine, d'arginine et de lysine. De préférence, le ou les sels de l'acide ellagique et/ou de ses éthers utilisables selon l'invention sont choisis parmi les sels des métaux alcalins ou alcalino-terreux, notamment les sels de sodium, de potassium, de calcium ou de magnésium.
11) D'autres agents antipelliculaires sont le soufre sous ses différentes formes, le sulfure de cadmium, l'allantoïne, les goudrons de houille ou de bois et leurs dérivés en particulier l'huile de cade, l'acide undécylénique, l'acide fumarique, et les allylamines telle que la terbinafine.

Préférentiellement, la composition selon l'invention comprend un ou plusieurs agents antipelliculaires choisis parmi la zinc pyrithione, la piroctone olamine, le sulfure de sélénium, l'acide ellagique et leurs mélanges.

Les agents antipelliculaires sont de préférence présents dans la composition en une quantité allant de 0,01 à 10% en poids, préférentiellement de 0,2 à 5% en poids, mieux encore de 0,5 à 3% en poids, par rapport au poids total de la composition.

### Polyols

La composition selon l'invention peut en outre comprendre au moins un polyol; elle peut bien évidemment comprendre plusieurs polyols.
De préférence, le ou les polyols de l'invention ne sont pas des polysaccharides.

Les polyols susceptibles d'être utilisés dans le cadre de la présente invention sont de préférence de formule : 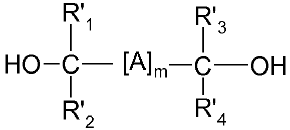 dans laquelle :
- R'₁, R'₂, R'₃, R'₄ désignent, indépendamment l'un de l'autre, un atome d'hydrogène, un radical alkyle en C₁-C₆ ou un radical mono ou polyhydroxyalkyle en C₁-C₆,
- A désigne un radical alkylène linéaire ou ramifié comprenant de 1 à 18 atomes de carbone, et éventuellement de 1 à 9 atomes d'oxygène, mais pas de groupement hydroxyle,
- m désigne 0 ou 1.

Un premier groupe de polyols préférés est constitué des polyols de formule ci-dessus pour laquelle m=0 tels que le 1,2,3-propanetriol (glycérine), le propylène glycol (ou 1,2-propanediol), le pinacol (2,3-diméthyl 2,3-butanediol), le 1,2,3-butanetriol, le 2,3-butanediol, l'octane1-2 diol et le sorbitol.

Un deuxième groupe de polyols préférés est constitué des polyols de formule ci-dessus pour laquelle m=1 et R'₁, R'₂, R'₃, R'₄ désignent, indépendamment l'un de l'autre, un atome d'hydrogène ou un radical alkyle en C₁-C₆; tels que les polyéthylèneglycols notamment ceux ayant de 4 à 9 groupement oxyde d'éthylène tels que par exemple les produits nommés PEG-6 ou PEG-8 (nom CTFA).

Un troisième groupe de polyols préférés est constitué des polyols de formule ci-dessus pour laquelle m=1 et R'₁, R'₂, R'₃, R'₄ désignent, indépendamment l'un de l'autre, un atome d'hydrogène, un radical alkyle en C₁-C₆ ou un radical mono ou polyhydroxyalkyle en C₁-C₆, et A désigne un radical alkylène linéaire ou ramifié comprenant 1 à 6 atomes de carbone; tels que le 3-méthyl-1,3,5-pentanetriol, le 1,2,4-butanetriol, le 1,5-pentanediol, le 2-méthyl-1,3 propanediol, le 1,3-butanediol, le 3-méthyl-1,5-pentanediol, le néopentylglycol (2,2-diméthyl-1,3-propanediol), l'isoprène glycol (3-méthyl-1,3-butanediol) et l'hexylèneglycol (2-méthyl-2,4-pentanediol) et de manière encore plus préférée l'hexylèneglycol, le propylèneglycol, le néopentylglycol et le 3-méthyl-1,5-pentanediol.

De préférence, les polyols employés sont liquides à 25°C, 1 atm.

De préférence, la composition selon l'invention comprend un ou plusieurs polyols plus particulièrement choisis parmi la glycérine, le propylène glycol, le sorbitol, les polyéthylèneglycols, l'hexylèneglycol et leurs mélanges.

Les polyols sont de préférence présents dans la composition en une quantité allant de 0,1 à 60% en poids, préférentiellement de 0,5 à 50% en poids, mieux encore de 1 à 30% en poids, par rapport au poids total de la composition.

### Ingrédients additionnels

La composition selon l'invention peut comprendre en outre, d'autres ingrédients utilisés couramment dans les compositions cosmétiques. De tels ingrédients peuvent être choisis parmi les antioxydants, les parfums, les huiles essentielles, les conservateurs, les actifs cosmétiques, les hydratants, les vitamines, les filtres solaires, les émulsionnants, les épaississants, les gélifiants, les agents d'étalement, les agents mouillants, les agents dispersants, les antimousses, les neutralisants, les stabilisants, et leurs mélanges. Bien entendu, l'homme du métier veillera à choisir ces éventuels ingrédients complémentaires et/ou leur quantité, de manière telle que les propriétés avantageuses de la composition selon l'invention ne soient pas, ou substantiellement pas, altérées par l'adjonction envisagée.

La composition selon l'invention peut comprendre en outre un ou plusieurs tensioactifs amphotères ou zwittérioniques.
Lesdits tensioactifs amphotères ou zwittérioniques additionnels susceptibles d'être utilisés dans l'invention peuvent être des dérivés d'amines aliphatiques secondaire ou tertiaire, éventuellement quaternisées, dans lesquels le groupe aliphatique est une chaîne linéaire ou ramifiée comportant 8 à 22 atomes de carbone, lesdits dérivés d'amines contenant au moins un groupe anionique tel que, par exemple, un groupe carboxylate, sulfonate, sulfate, phosphate ou phosphonate.

On peut citer en particulier les alkyl(C8-C20)bétaïnes, les sulfobétaïnes, les alkyl(C₈-C₂₀)sulfobétaïnes, les alkyl(C8-C20)amidoalkyl(C1-C6)bétaïnes telles que la cocoamidopropylbétaïne, les alkyl(C8-C20)amidoalkyl(C1-C6)sulfobétaïnes.

Parmi les dérivés d'amines aliphatiques secondaires ou tertiaires éventuellement quaternisées susceptibles d'être employés, on peut également citer les produits de structures respectives (A2) et (A3) suivantes :

Rₐ-CONHCH₂CH₂-N⁺(R_{b})(R_{c})(CH₂COO⁻) (A2)

dans laquelle :
Rₐ représente un groupe alkyle ou alkényle en C₁₀-C₃₀ dérivé d'un acide Rₐ-COOH de préférence présent dans l'huile de coprah hydrolysée, un groupe heptyle, nonyle ou undécyle,
R_{b} représente un groupe bêta-hydroxyéthyle, et
R_{c} représente un groupe carboxyméthyle ;

R_{a'}-CONHCH₂CH₂-N(B)(B') (A3)

dans laquelle :
B représente -CH₂CH₂OX',
X' représente le groupe -CH₂-COOH, CH₂-COOZ', -CH₂CH₂-COOH, -CH₂CH₂-COOZ', ou un atome d'hydrogène,
B' représente -(CH₂)_{z}-Y', avec z = 1 ou 2,
Y' représente -COOH, -COOZ', le groupe -CH₂-CHOH-SO₃H ou -CH₂-CHOH-SO₃Z',
Z' représente un ion issu d'un métal alcalin ou alcalinoterreux, tel que le sodium, le potassium ou le magnésium; un ion ammonium; ou un ion issu d'une amine organique et notamment d'un aminoalcool, tel que la mono-, di- et triéthanolamine, la mono-, di- ou tri-isopropanol-amine, le 2-amino 2-méthyl 1-propanol, le 2-amino 2-méthyl 1,3-propanediol et le tris(hydroxyméthyl)amino méthane.
R_{a'} représente un groupe alkyle ou alkényle en C₁₀-C₃₀ d'un acide R_{a'}COOH de préférence présent dans l'huile de coprah ou dans l'huile de lin hydrolysée, un groupe alkyle, notamment en C₁₇ et sa forme iso, un groupe en C₁₇ insaturé.

Les composés répondant à la formule (A3) sont préférés. Ces composés sont également classés dans le dictionnaire CTFA, 5^{ème} édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.
A titre d'exemple, on peut citer le cocoamphodiacétate commercialisé par la société RHODIA sous la dénomination commerciale MIRANOL® C2M concentré.

On peut aussi utiliser des composés de formule (A4) :

R_{a"}-NH-CH(Y")-(CH₂)n-C(O)-NH-(CH₂)n'-N(R_{d})(Rₑ) (A4)

dans laquelle :
- Rₐ" représente un groupe alkyle ou alcényle en C₁₀-C₃₀ d'un acide Rₐ"-C(O)OH, de préférence présent dans l'huile de coprah ou dans l'huile de lin hydrolysée;
- Y" représente le groupe -C(O)OH, -C(O)OZ", -CH₂-CH(OH)-SO₃H ou le groupe -CH₂-CH(OH)-SO₃-Z" avec Z" représentant un contre ion cationique issu d'un métal alcalin ou alcalinoterreux, tel que le sodium, un ion ammonium ou un ion issu d'une amine organique;
- R_{d} et Rₑ, indépendamment l'un de l'autre, représentent un radical alkyle ou hydroxyalkyle en C1-C4; et
- n et n', indépendamment l'un de l'autre, désignent un nombre entier allant de 1 à 3.

On peut notamment citer le composé classé dans le dictionnaire CTFA sous la dénomination sodium diethylaminopropyl cocoaspartamide et commercialisé par la société CHIMEX sous l'appellation CHIMEXANE HB.
De préférence, les tensioactifs amphotères ou zwittérioniques sont choisis parmi les alkyl(C8-C20)bétaïnes, les alkyl(C8-C20)amidoalkyl(C1-C6)bétaïnes et les alkyl(C8-C20) amphodiacétates, ainsi que le sel de sodium du laurylaminosuccinamate de diéthylaminopropyle; et leurs mélanges.
Préférentiellement, les tensioactifs amphotères ou zwittérioniques sont choisis parmi, seul ou en mélange, la cocoylamidopropylbétaïne, la cocoylbétaïne et le cocoamphodiacétate.

La composition selon l'invention peut comprendre le ou lesdits tensioactifs amphotères ou zwittérioniques en une quantité de préférence comprise entre 0,1 et 30% en poids, notamment entre 1 et 20% en poids, par rapport au poids total de la composition.

La composition peut en outre comprendre un ou plusieurs sels solubles dans ladite composition, lesdits sels étant différents d'un tensioactif.
Lesdits sels sont de préférence hydrosolubles et présentent une solubilité dans l'eau supérieure à 5% à 25°C, 1 atm. Préférentiellement, on utilise un sel d'un métal alcalin ou alcalinoterreux, encore plus préférentiellement un sel minéral. On peut notamment citer le chlorure de sodium et le chlorure de magnésium.
De préférence, lesdits sels hydrosolubles sont présents dans la composition de l'invention à une concentration allant de 0,01 à 20% en poids, mieux de 0,1 à 10% en poids, par rapport au poids total de la composition.

La composition selon l'invention n'est pas anhydre; elle est aqueuse ce qui signifie qu'elle comprend au moins 20% en poids d'eau; de préférence elle comprend de l'eau à une concentration allant de 20 à 80% en poids, notamment de 35 à 75% en poids, voire de 40 à 70% en poids, mieux de 45 à 65% en poids, encore mieux de 50 à 65% en poids, par rapport au poids total de la composition.
La composition peut également comprendre en outre un ou plusieurs solvants organiques liquides à 25°C, 1 atm., différents des composés cités ci-dessus, notamment les polyols et/ou conditionneurs, tels que les alcools en C1-C7, notamment les monoalcools aliphatiques ou aromatiques en C1-C7, tels que l'éthanol, l'isopropanol, l'alcool benzylique et leurs mélanges.

De préférence, la composition présente un pH compris entre 2 et 11, notamment 3 à 9, préférentiellement compris entre 5 et 7.

Les compositions selon l'invention peuvent être préparées par mélange des différents ingrédients à température ambiante (25°C), ou bien par mélange à chaud, à une température comprise entre 25 et 80°C d'une phase aqueuse et d'une phase grasse, en particulier en cas de présence de corps gras solides.

La composition cosmétique selon l'invention trouve notamment une application particulièrement intéressante dans le domaine de l'hygiène capillaire, notamment pour le soin, le nettoyage et/ou le conditionnement des matières kératiniques, en particulier le nettoyage des cheveux.
Les compositions capillaires sont de préférence des shampooings, des compositions antichute, ou antiparasitaire, ou antipelliculaire ou antiséborrhéique.

La composition cosmétique peut être rincée ou non après avoir été appliquée sur les matières kératiniques. On peut ainsi effectuer optionnellement un rinçage par exemple avec de l'eau après un éventuel temps de pose. De préférence, la composition est rincée.

L'invention a également pour objet un procédé de traitement cosmétique, notamment de soin, de nettoyage et/ou de conditionnement des matières kératiniques, qui consiste à appliquer une composition telle que décrite ci-dessus, sur lesdites matières kératiniques, à effectuer optionnellement un rinçage par exemple avec de l'eau après un éventuel temps de pose. De préférence, on effectue un rinçage après un éventuel temps de pose.
Il s'agit de préférence d'un procédé de traitement capillaire, pour le nettoyage ou lavage des cheveux.

La présente invention est illustrée plus en détails dans les exemples qui suivent (% MA = % de matière active dans la composition).

### Exemples 1 à 4

On prépare des compositions capillaires de lavage comprenant les ingrédients suivants (% en poids de matière première commerciale) :

| | **Exemple 1** | **Exemple 2** | **Exemple 3** | **Exemple 4** |
|---|---|---|---|---|
| SODIUM COCOYL ISETHIONATE (HOSTAPON SCI 85) | | 25 (22% MA) | 33 (29% MA) | 25 (22% MA) |
| COCOYL ISETHIONATE DE SODIUM / ISETHIONATE DE SODIUM (JORDAPON CI de BASF) | 33,6% (28,7% MA) | | | |
| Lauryléthersulfate de sodium (2.2 OE) en solution aqueuse à 70% MA | | | | 5 (3,5% MA) |
| Cocoyl amidopropylbétaïne en solution aqueuse à 38% MA | | 6 (2,3% MA) | | |
| Terpolymère acide acrylique/ MAPTAC / acrylamide à 20% en solution aqueuse (Merquat 2003) | 0,4 (0,08% MA) | | | 0,4 (0,08% MA) |
| Chlorure d'hydroxypropylguar triméthylammonium | | | 0,2 | |
| Glycérine oxyéthylénée (26 OE) | 3,7 | 4 | 3,7 | 4 |
| Chlorure de polydiméthyl diallyl ammonium à 40% dans l'eau (Polyquaternium 6) | | 1 (0,4% MA) | | |
| Amodiméthicone à 57,5% MA (Xiameter MEM-8299 | | | | 2,4 (1,38% MA) |
| Emulsion de Dow Corning) | | | | |
| Distéarate de glycol | 6,7% | | 6,7% | |
| Octane-1,2 diol | 0.3 | | 0.3 | |
| Parfum, conservateur | Qs | Qs | Qs | Qs |
| Agent de pH | Qsp pH 5.5 | Qsp pH 5.5 | Qsp pH 5.5 | Qsp pH 5.5 |
| Eau | Qsp 100% | Qsp 100% | Qsp 100% | Qsp 100% |

Les compositions selon les exemples se présentent sous forme de solides souples.

Ces compositions sont employées pour le nettoyage des cheveux. Lors de l'utilisation, on constate une bonne répartition du produit sur la chevelure, ainsi que l'obtention d'une mousse crémeuse, onctueuse et abondante. Les compositions se rincent et s'éliminent facilement.

On teste la composition de l'exemple 3 sur demi-tête, en comparaison avec un shampoing classique, sur un panel de 6 personnes.
On constate qu'avec la composition selon l'invention :
- la répartition du produit et le démarrage de la mousse sont proches de ceux obtenus avec le shampoing classique;
- l'abondance de mousse est nettement supérieure; les cheveux sont plus lisses dans la mousse;
- la rapidité de rinçage est très nettement supérieure.
Avec la composition selon l'invention, la souplesse des cheveux mouillés est améliorée, les cheveux sont plus individualisés et plus toniques. Sur cheveux secs, les cheveux sont plus toniques.

Pour l'exemple 3:
- on trace le spectre viscoélastique (G' et G" en fonction de la fréquence), pour des fréquences comprises entre 10⁻² Hz et 100 Hz, à 25°C, dans les conditions décrites dans la description; on constate qu'il n'y a aucun point de croisement des deux courbes.
- on mesure également la contrainte de seuil à 25°C, et on détermine que cette contrainte est supérieure à 100 Pa.
- la force de pénétration est mesurée et vaut 427 g.

### Exemples 5 à 8

On prépare des compositions capillaires de lavage comprenant les ingrédients suivants (% en poids de matière première commerciale) :

| | **Exemple 5** | **Exemple 6** | **Exemple 7** | **Exemple 8** |
|---|---|---|---|---|
| SODIUM COCOYL | 33 | 33 | 33 | 33 |

| ISETHIONATE (HOSTAPON SCI 85) | (29% MA) | (29% MA) | (29% MA) | (29% MA) |
|---|---|---|---|---|
| Kaolin | | | | 3 |
| PDMS (500 000 cst; MW = 250 000) | | 2 | 2 | 2 |
| Chlorure de polydiméthyl diallyl ammonium à 40% dans l'eau (Polyquaternium 6) | 1 (0,4% MA) | | | |
| Amodiméthicone à 57,5% MA (Xiameter MEM-8299 Emulsion de Dow Corning) | 2% MA | | | |
| Glycérine | 15 | 20 | 20 | 20 |
| Distéarate de glycol | 6,7% | 6,7% | 6,7% | 6,7% |
| NaCl | 3,5 | 3,5 | 3,5 | 3,5 |
| Pyrithione de zinc (à 48% dans l'eau) | | | 2,8% (1% MA) | |
| Monolaurate de sorbitane 4 OE | | 3% | | |
| Octane-1,2 diol | 0.2 | | | |
| Parfum, conservateur | Qs | Qs | Qs | Qs |
| Agent de pH | Qsp pH 5.5 | Qsp pH 5.5 | Qsp pH 5.5 | Qsp pH 5.5 |
| Eau | Qsp 100% | Qsp 100% | Qsp 100% | Qsp 100% |

Les compositions 5 à 8 se présentent sous forme de solides souples.
Ces compositions sont employées pour le nettoyage des cheveux. Lors de l'utilisation, on constate une bonne répartition du produit sur la chevelure, ainsi que l'obtention d'une mousse crémeuse, onctueuse et abondante. Les compositions se rincent et s'éliminent facilement.

## Revendications

1. Composition cosmétique aqueuse se présentant sous forme de solide souple, comprenant :
(i) un ou plusieurs tensioactifs anioniques, en une quantité totale variant de 15 à 60% en poids par rapport au poids total de la composition,
ladite composition comprenant au moins un tensioactif anionique choisi parmi les acyliséthionates, de préférence ayant un groupe acyle comportant de 6 à 30 atomes de carbone, mieux de 12 à 24, encore mieux de 16 à 22 atomes de carbone;
la quantité totale en acyliséthionates dans la composition variant de 12 à 35% en poids par rapport au poids total de la composition ;
(ii) un ou plusieurs agents conditionneurs polymériques choisis parmi les polymères cationiques non siliconés, les polymères amphotères non siliconés, les silicones, notamment aminées, et leurs mélanges ;
ladite composition comprenant au moins 20% en poids d'eau par rapport au poids total de la composition ; et
ladite composition présentant une contrainte de seuil à 25°C supérieure ou égale à 100 Pa, et/ou présentant une force de pénétration à 25°C supérieure ou égale à 210 g, et/ou présentant un spectre viscoélastique à 25°C, mesuré entre 10⁻² Hz et 100 Hz, tel qu'il n'y a pas de point de croisement entre les courbes G' et G"; G' étant toujours strictement supérieur à G".

2. Composition selon la revendication 1, présentant une contrainte de seuil à 25°C allant de 100 à 900 Pa.

3. Composition selon l'une des revendications précédentes, présentant une force de pénétration à 25°C allant de 210 à 900 g, mieux allant de 350 à 800 g.

4. Composition selon l'une des revendications précédentes, dans laquelle les tensioactifs acyliséthionates répondent à la formule R-C(O)-O-CH₂CH₂SO₃M, dans laquelle R-C(O) est un groupe acyle comportant de préférence de 6 à 30 atomes de carbone, mieux de 12 à 24, encore mieux de 16 à 22 atomes de carbone, et M désigne un contre-ion cosmétiquement acceptable; de préférence sont choisis parmi les cocoyl-iséthionates et/ou les lauroyl-iséthionates.

5. Composition selon l'une des revendications précédentes, comprenant un ou plusieurs tensioactifs anioniques additionnels sulfate(s), sulfonate(s) ou carboxylate(s), de préférence choisis parmi :
- les alkylsulfates en C6-C24, notamment en C12-C20,
- les alkyléthersulfates en C6-C24, notamment en C12-C20; comprenant de préférence de 2 à 20 motifs oxyde d'éthylène,
- les acylglutamates en C6-C24, notamment en C12-C20; notamment les stéaroylglutamates;
- les acylsarcosinates en C6-C24, notamment en C12-C20; notamment les palmitoylsarcosinates;
- les acyllactylates en C6-C24, notamment en C12-C20; notamment les béhénoyllactylates;
- les alkyl(C6-C24)éthercarboxylates, de préférence les alkyl(C12-C20)éthercarboxylates;
- les alkylsulfosuccinates en C6-C24, notamment en C12-C20, notamment les laurylsulfosuccinates.

6. Composition selon l'une des revendications précédentes, dans laquelle la quantité totale de tensioactif(s) anionique(s) varie de 20 à 60% en poids, préférentiellement de 25 à 60% en poids, voire de 30 à 60% en poids, par rapport au poids total de la composition.

7. Composition selon l'une des revendications précédentes, dans laquelle la quantité totale d'acyliséthionates dans la composition varie de 15 à 35% en poids, encore mieux de 20 à 35% en poids, par rapport au poids total de la composition.

8. Composition selon l'une des revendications précédentes, dans laquelle les agents conditionneurs polymériques sont choisis parmi les polymères cationiques non siliconés et les silicones, notamment aminées; et encore mieux leurs mélanges.

9. Composition selon l'une des revendications précédentes, dans laquelle les agents conditionneurs polymériques sont choisis parmi :
(a) les homopolymères ou copolymères dérivés d'esters ou d'amides acryliques ou méthacryliques et comportant au moins un des motifs de formule suivante : 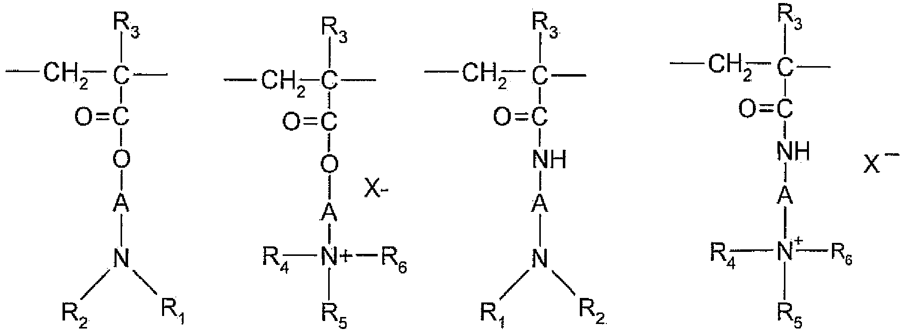 dans lesquelles:
- R3, identiques ou différents, désignent un atome d'hydrogène ou un radical CH3;
- A, identiques ou différents, représentent un groupe divalent alkyle, linéaire ou ramifié, de 1 à 6 atomes de carbone, de préférence 2 ou 3 atomes de carbone ou un groupe hydroxyalkyle de 1 à 4 atomes de carbone ;
- R4, R5, R6, identiques ou différents, représentent un groupe alkyle ayant de 1 à 18 atomes de carbone ou un radical benzyle; de préférence un groupe alkyle ayant de 1 à 6 atomes de carbone;
- R1 et R2, identiques ou différents, représentent un atome d'hydrogène ou un groupe alkyle ayant de 1 à 6 atomes de carbone, de préférence méthyle ou éthyle;
- X désigne un anion dérivé d'un acide minéral ou organique tel qu'un anion méthosulfate ou un halogénure tel que chlorure ou bromure.
(b) Les polysaccharides cationiques, notamment les celluloses et les gommes de galactomannanes cationiques.
(c) les cyclopolymères d'alkyl diallyl amine ou de dialkyl diallyl ammonium tels que les homopolymères ou copolymères comportant comme constituant principal de la chaîne des motifs répondant aux formules (I) ou (II) : 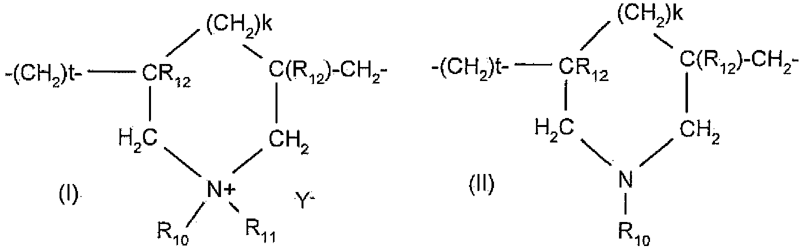 dans lesquelles
- k et t sont égaux à 0 ou 1, la somme k + t étant égale à 1 ;
- R12 désigne un atome d'hydrogène ou un radical méthyle ;
- R10 et R11, indépendamment l'un de l'autre, désignent un groupement alkyle ayant de 1 à 6 atomes de carbone, un groupement hydroxyalkyle dans lequel le groupement alkyle a 1 à 5 atomes de carbone, un groupement amidoalkyle en C1-C4; ou bien R10 et R11 peuvent désigner conjointement avec l'atome d'azote auquel ils sont rattachés, des groupement hétérocycliques, tels que pipéridinyle ou morpholinyle ; R10 et R11, indépendamment l'un de l'autre, désignent de préférence un groupement alkyle ayant de 1 à 4 atomes de carbone.
- Y⁻ est un anion tel que bromure, chlorure, acétate, borate, citrate, tartrate, bisulfate, bisulfite, sulfate, phosphate.
(d) Les polymères quaternaires de vinylpyrrolidone et de vinylimidazole;
(e) les polydialkylsiloxanes, notamment les polydiméthylsiloxanes (PDMS),
(f) les silicones aminées, et notamment répondant à la formule suivante : 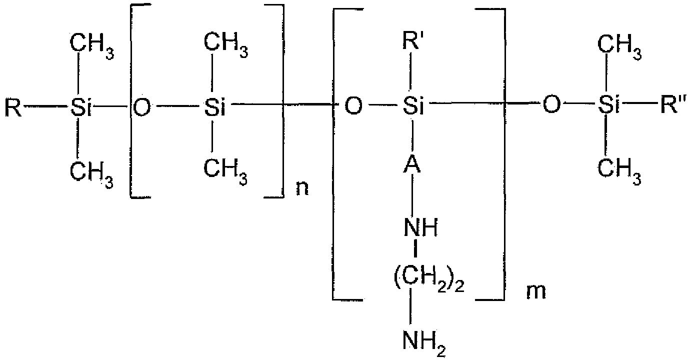 dans laquelle R, R', R", identiques ou différents, désignent un radical alkyle en C₁-C₄, de préférence CH₃; un radical alcoxy en C₁-C₄, de préférence méthoxy; ou OH ; A représente un radical alkylène, linéaire ou ramifié, en C1-C8, de préférence en C₃-C₆; m et n sont des nombres entiers dépendant du poids moléculaire et dont la somme est comprise entre 1 et 2000.

10. Composition selon l'une des revendications précédentes, dans laquelle les agents conditionneurs polymériques sont présents en une quantité allant de 0,001 à 20% en poids, préférentiellement de 0,1 à 10% en poids, mieux encore de 0,2 à 8% en poids, voire de 0,4 à 5% en poids, préférentiellement de 0,5 à 5% en poids, par rapport au poids total de la composition.

11. Composition selon l'une des revendications précédentes, comprenant en outre un ou plusieurs agents conditionneurs non polymériques, qui peuvent être choisis parmi les tensioactifs cationiques, les esters gras autres que les triglycérides, les alcools gras, les huiles végétales, les hydrocarbures liquides en C6-C16, les hydrocarbures ayant plus de 16 atomes de carbone, les céramides, les cires végétales ou animales, et leurs mélanges.

12. Composition selon l'une des revendications précédentes, comprenant en outre un ou plusieurs types de particules solides, généralement insolubles dans l'eau; de préférence choisies parmi :
- les particules de noyaux de fruits micronisés;
- les sels de pyridinethione, notamment les sels de calcium, de magnésium, de baryum, de strontium, de zinc, de cadmium, d'étain et de zirconium;
- les particules de polymères réticulés ou non; et notamment les poudres de polyamide; les poudres de polymères acryliques, notamment de polyacrylate de sodium réticulé, de polyméthacrylate de méthyle; les poudres de copolymères acryliques, notamment de polyméthacrylate de méthyle/diméthacrylate d'éthylène glycol, de polyméthacrylate d'allyle/diméthacrylate d'éthylène glycol, de copolymère diméthacrylate d'éthylène glycol/méthacrylate de lauryle, de polyacrylate/alkylacrylate; les poudres de polystyrène; les poudres de polyéthylène, notamment de polyéthylène/acide acrylique; les microbilles de résine de silicone;
- les particules métalliques, les oxydes, les sels inorganiques, les carbures, les nitrures, les borures, les sulfures et les hydroxydes; et notamment les argiles, les silicates, l'alumine, la silice, le kaolin et l'hydroxyapatite;
- les pigments colorés organiques ou minéraux, ou encore les agents nacrants non colorés;
lesdites particules solides étant de préférence présentes en une quantité allant de 0,001 à 15% en poids, préférentiellement de 0,1 à 10% en poids, mieux encore de 0,5 à 8% en poids, par rapport au poids total de la composition.

13. Composition selon l'une des revendications précédentes, comprenant en outre un ou plusieurs polyols; de préférence de formule : 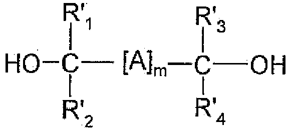 dans laquelle :
- R'₁, R'₂, R'₃, R'₄ désignent, indépendamment l'un de l'autre, un atome d'hydrogène, un radical alkyle en C₁-C₆ ou un radical mono ou polyhydroxyalkyle en C₁-C₆,
- A désigne un radical alkylène linéaire ou ramifié comprenant de 1 à 18 atomes de carbone, et éventuellement de 1 à 9 atomes d'oxygène, mais pas de groupement hydroxyle,
- m désigne 0 ou 1;
de préférence, lesdits polyols étant présents en une quantité allant de 0,1 à 60% en poids, préférentiellement de 0,5 à 50% en poids, mieux encore de 1 à 30% en poids, par rapport au poids total de la composition.

14. Composition selon l'une des revendications précédentes, comprenant en outre un ou plusieurs agents antipelliculaires; notamment choisis parmi :
- les sels de pyridinethione ;
- les dérivés de 1-hydroxy-2-pyrrolidone ;
- le 2,2'-dithio-bis-(pyridine-N-oxide) ;
- les trihalogénocarbamides ;
- le triclosan ;
- les composés azolés ;
- les polymères antifongiques ;
- les sulfures de sélénium ;
- les extraits d'une ou plusieurs bactéries filamenteuses non photosynthétiques non fructifiantes;
- l'acide ellagique, ses éthers, les sels de l'acide ellagique et les sels de ses éthers; ainsi que les tannins d'acide ellagique;
- le soufre, le sulfure de cadmium, l'allantoïne, les goudrons de houille ou de bois et leurs dérivés, l'acide undécylénique, l'acide fumarique, et les allylamines telle que la terbinafine;
de préférence, lesdits agents antipelliculaires étant présents en une quantité allant de 0,01 à 10% en poids, préférentiellement de 0,2 à 5% en poids, mieux encore de 0,5 à 3% en poids, par rapport au poids total de la composition.

15. Composition selon l'une des revendications précédentes, comprenant de l'eau à une concentration allant de 20 à 80% en poids, notamment de 35 à 75% en poids, voire de 40 à 70% en poids, mieux de 45 à 65% en poids, encore mieux de 50 à 65% en poids, par rapport au poids total de la composition.

16. Procédé de traitement cosmétique, notamment de soin, de nettoyage et/ou de conditionnement des matières kératiniques, notamment des cheveux, qui consiste à appliquer une composition telle que définie à l'une des revendications 1 à 15, sur lesdites matières kératiniques, à effectuer optionnellement un rinçage par exemple avec de l'eau après un éventuel temps de pose.

## Patentansprüche

1. Wässrige kosmetische Zusammensetzung, die in flexibler fester Form vorliegt, umfassend:
(i) ein oder mehrere anionische Tenside in einer Gesamtmenge im Bereich von 15 bis 60 Gew.-%, bezogen auf das Gesamtgewicht der Zusammensetzung, wobei die Zusammensetzung mindestens ein anionisches Tensid umfasst, das aus Acylisethionaten, die vorzugsweise eine Acylgruppe mit 6 bis 30 Kohlenstoffatomen, besser 12 bis 24 und noch besser 16 bis 22 Kohlenstoffatomen umfassen, ausgewählt ist;
wobei die Gesamtmenge an Acylisethioanaten in der Zusammensetzung im Bereich von 12 bis 35 Gew.-%, bezogen auf das Gesamtgewicht der Zusammensetzung, liegt;
(ii) ein oder mehrere polymere Konditionierungsmittel, die aus kationischen Nichtsilikonpolymeren, amphoteren Nichtsilikonpolymeren, Silikonen, insbesondere Aminosilikonen, und Mischungen davon ausgewählt sind;
wobei die Zusammensetzung mindestens 20 Gew.-% Wasser, bezogen auf das Gesamtgewicht der Zusammensetzung, umfasst und
wobei die Zusammensetzung eine Grenzspannung bei 25 °C größer oder gleich 100 Pa aufweist und/oder eine Penetrationskraft bei 25 °C größer oder gleich 210 g aufweist und/oder ein solches zwischen 10⁻² Hz und 100 Hz gemessenes viskoelastisches Spektrum bei 25 °C aufweist, dass es keinen Kreuzungspunkt zwischen den Kurven G' und G" gibt; wobei G' immer streng größer als G" ist.

2. Zusammensetzung nach Anspruch 1 mit einer Grenzspannung bei 25 °C im Bereich von 100 bis 900 Pa.

3. Zusammensetzung nach einem der vorhergehenden Ansprüche mit einer Penetrationskraft bei 25 °C im Bereich von 210 bis 900 g, besser im Bereich von 350 bis 800 g.

4. Zusammensetzung nach einem der vorhergehenden Ansprüche, wobei die Acylisethionat-Tenside der Formel R-C(O)-O-CH₂CH₂SO₃M entsprechen, wobei R-C(O) für eine Acylgruppe mit vorzugsweise 6 bis 30 Kohlenstoffatomen, besser 12 bis 24, noch besser 16 bis 22 Kohlenstoffatomen steht und M ein kosmetisch unbedenkliches Gegenion bedeutet; vorzugsweise aus Cocoylisethionaten und/oder Lauroylisethionaten ausgewählt sind.

5. Zusammensetzung nach einem der vorhergehenden Ansprüche, umfassend ein oder mehrere zusätzliche anionische Sulfat-, Sulfonat- oder Carboxylat-Tenside, vorzugsweise ausgewählt aus:
- C6-C24- und insbesondere C12-C20-Alkylsulfaten;
- C6-C24- und insbesondere C12-C20-Alkylethersulfaten, vorzugsweise mit 2 bis 20 Ethylenoxideinheiten;
- C6-C24- und insbesondere C12-C20-Acylglutamaten; insbesondere Stearoylglutamaten;
- C6-C24- und insbesondere C12-C20-Acylsarkosinaten; insbesondere Palmitoylsarkosinaten;
- C6-C24- und insbesondere C12-C20-Acyllactylaten; insbesondere Behenoyllactylaten;
- (C6-C24)-Alkylethercarboxylaten, vorzugsweise (C12-C20)-Alkylethercarboxylaten;
- C6-C24- und insbesondere C12-C20-Alkylsulfosuccinaten, insbesondere Laurylsulfosuccinaten.

6. Zusammensetzung nach einem der vorhergehenden Ansprüche, wobei die Gesamtmenge an anionischem Tensid bzw. anionischen Tensiden im Bereich von 20 bis 60 Gew.-%, vorzugsweise 25 bis 60 Gew.-% oder sogar 30 bis 60 Gew.-%, bezogen auf das Gesamtgewicht der Zusammensetzung, liegt.

7. Zusammensetzung nach einem der vorhergehenden Ansprüche, wobei die Gesamtmenge an Acylisethionaten in der Zusammensetzung im Bereich von 15 bis 35 Gew.-%, noch besser von 20 bis 35 Gew.-%, bezogen auf das Gesamtgewicht der Zusammensetzung, variiert.

8. Zusammensetzung nach einem der vorhergehenden Ansprüche, wobei die polymeren Konditionierungsmittel aus kationischen Nichtsilikonpolymeren und Silikonen, insbesondere Aminosilikonen, und noch besser Mischungen davon ausgewählt sind.

9. Zusammensetzung nach einem der vorhergehenden Ansprüche, wobei die polymeren Konditionierungsmittel ausgewählt sind aus:
(a) Homopolymeren oder Copolymeren, die sich von Acrylsäure- oder Methacrylsäureestern oder -amiden ableiten und mindestens eine der Einheiten der folgenden Formeln umfassen: 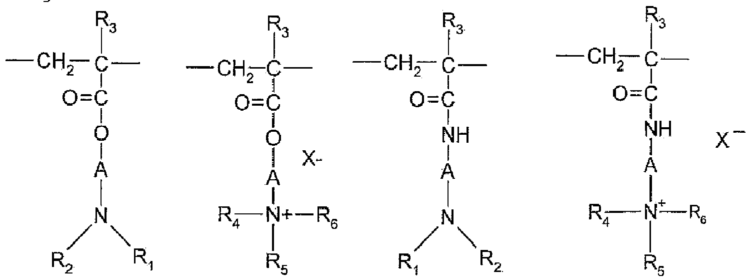 in denen:
- die Variablen R3, die gleich oder verschieden sind, für ein Wasserstoffatom oder einen CH3-Rest stehen;
- die Variablen A, die gleich oder verschieden sind, für eine lineare oder verzweigte zweiwertige Alkylgruppe mit 1 bis 6 Kohlenstoffatomen, vorzugsweise 2 oder 3 Kohlenstoffatomen, oder eine Hydroxyalkylgruppe mit 1 bis 4 Kohlenstoffatomen stehen;
- R4, R5 und R6, die gleich oder verschieden sind, für eine Alkylgruppe mit 1 bis 18 Kohlenstoffatomen oder einen Benzylrest, vorzugsweise eine Alkylgruppe mit 1 bis 6 Kohlenstoffatomen, stehen;
- R1 und R2, die gleich oder verschieden sind, für ein Wasserstoffatom oder eine Alkylgruppe mit 1 bis 6 Kohlenstoffatomen, vorzugsweise Methyl oder Ethyl, stehen;
- X für ein Anion steht, das sich von einer anorganischen oder organischen Säure ableitet, wie ein Methosulfat-Anion oder ein Halogenid wie Chlorid oder Bromid, steht;
(b) kationischen Polysacchariden, insbesondere Cellulosen und kationischen Galactomannangummen;
(c) Cyclopolymeren von Alkyldiallylamin oder Dialkyldiallylammonium wie Homopolymeren oder Copolymeren, die als Hauptbestandteil der Kette Einheiten umfassen, die den Formeln (I) oder (II) entsprechen: 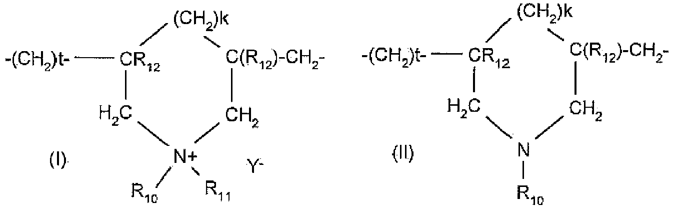 in denen
- k und t gleich 0 oder 1 sind, wobei die Summe k + t gleich 1 ist;
- R12 für ein Wasserstoffatom oder einen Methylrest steht;
- R10 und R11 unabhängig voneinander für eine Alkylgruppe mit 1 bis 6 Kohlenstoffatomen, eine Hydroxyalkylgruppe mit 1 bis 5 Kohlenstoffatomen in der Alkylgruppe oder eine C1-C4-Amidoalkyl-gruppe stehen oder auch R10 und R11 zusammen mit dem Stickstoffatom, an das sie gebunden sind, für heterocyclische Gruppen, wie Piperidinyl oder Morpholinyl, stehen können; R10 und R11 unabhängig voneinander vorzugsweise für eine Alkylgruppe mit 1 bis 4 Kohlenstoffatomen stehen;
- Y⁻ für ein Anion wie Bromid, Chlorid, Acetat, Borat, Citrat, Tartrat, Bisulfat, Bisulfit, Sulfat oder Phosphat steht;
(d) quaternären Vinylpyrrolidon- und Vinylimidazolpolymeren;
(e) Polydialkylsiloxanen, insbesondere Polydimethylsiloxanen (PDMS);
(f) Aminosilikonen, und insbesondere entsprechend der folgenden Formel: 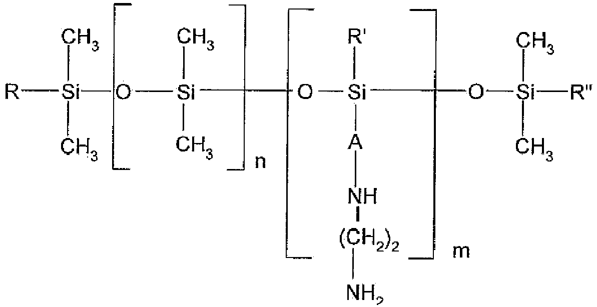 in der R, R' und R", die gleich oder verschieden sind, für einen C₁-C₄-Alkylrest, vorzugsweise CH₃, einen C₁-C₄-Alkoxyrest, vorzugsweise Methoxy, oder OH stehen; A für einen linearen oder verzweigten C₁-C₈- und vorzugsweise C₃-C₆-Alkylenrest steht; m und n für ganze Zahlen stehen, die vom Molekulargewicht abhängen und deren Summe zwischen 1 und 2000 liegt.

10. Zusammensetzung nach einem der vorhergehenden Ansprüche, wobei die polymeren Konditionierungsmittel in einer Menge im Bereich von 0,001 bis 20 Gew.-%, vorzugsweise von 0,1 bis 10 Gew.-%, noch besser von 0,2 bis 8 Gew.-% oder sogar 0,4 bis 5 Gew.-%, vorzugsweise von 0,5 bis 5 Gew.-%, bezogen auf das Gesamtgewicht der Zusammensetzung, vorliegen.

11. Zusammensetzung nach einem der vorhergehenden Ansprüche, außerdem umfassend ein oder mehrere nichtpolymere Konditionierungsmittel, die aus kationischen Tensiden, Fettestern, die von Triglyceriden verschieden sind, Fettalkoholen, pflanzlichen Ölen, flüssigen C6-16-Kohlenwasserstoffen, Kohlenwasserstoffen mit mehr als 16 Kohlenstoffatomen, Ceramiden, pflanzlichen oder tierischen Wachsen und Mischungen davon ausgewählt sein können.

12. Zusammensetzung nach einem der vorhergehenden Ansprüche, außerdem umfassend eine oder mehrere Arten von festen Teilchen, die im Allgemeinen in Wasser unlöslich sind; vorzugsweise ausgewählt aus:
- mikronisierten Fruchtkernteilchen;
- Pyridinthionsalzen, insbesondere den Calcium-, Magnesium-, Barium-, Strontium-, Zink-, Cadmium-, Zinn- und Zirconiumsalzen;
- Teilchen von vernetzten oder unvernetzten Polymeren und insbesondere Polyamidpulvern; Pulvern von Acrylpolymeren, insbesondere vernetztem Natriumpolyacrylat, Polymethylmethacrylat; Pulvern von Acrylcopolymeren, insbesondere Polymethylmethacrylat-ethylenglykoldimethacrylat, Polyallylmethacrylat-ethylenglykoldimethacrylat, Ethylenglykoldimethacrylat-Laurylmethacrylat-Copolymer, Polyacrylat-alkylacrylat; Polystyrolpulvern; Polyethylenpulvern, insbesondere Polyethylen-acrylsäure; Silikonharz-Mikrokügelchen;
- Metallteilchen, Oxiden, anorganischen Salzen, Carbiden, Nitriden, Boriden, Sulfiden und Hydroxiden und insbesondere Tonen, Silikaten, Aluminiumoxid, Siliciumdioxid, Kaolin und Hydroxyapatit;
- organischen oder anorganischen Farbpigmenten oder auch farblosen Perlglanzmitteln;
wobei die festen Teilchen vorzugsweise in einer Menge im Bereich von 0,001 bis 15 Gew.-%, vorzugsweise von 0,1 bis 10 Gew.-%, noch besser von 0,5 bis 8 Gew.-%, bezogen auf das Gesamtgewicht der Zusammensetzung, vorliegen.

13. Zusammensetzung nach einem der vorhergehenden Ansprüche, außerdem umfassend ein oder mehrere Polyole; vorzugsweise der Formel: 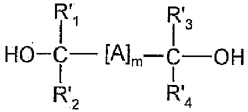 in der:
- R'₁, R'₂, R'₃ und R'₄ unabhängig voneinander für ein Wasserstoffatom, einen C₁-C₆-Alkylrest oder einen C₁-C₆-Mono- oder -Polyhydroxyalkylrest stehen,
- A für einen linearen oder verzweigten Alkylenrest mit 1 bis 18 Kohlenstoffatomen und gegebenenfalls 1 bis 9 Sauerstoffatomen, aber ohne Hydroxylgruppe, steht;
- m für 0 oder 1 steht;
wobei die Polyole vorzugsweise in einer Menge im Bereich von 0,1 bis 60 Gew.-%, vorzugsweise von 0,5 bis 50 Gew.-%, noch besser von 1 bis 30 Gew.-%, bezogen auf das Gesamtgewicht der Zusammensetzung, vorliegen.

14. Zusammensetzung nach einem der vorhergehenden Ansprüche, außerdem umfassend ein oder mehrere Antischuppenmittel; insbesondere ausgewählt aus:
- Pyridinthionsalzen;
- 1-Hydroxy-2-pyrrolidin-Derivaten;
- 2,2'-Dithiobis(pyridin-N-oxid);
- Trihalogencarbamiden;
- Triclosan;
- Azolverbindungen;
- antimykotischen Polymeren;
- Selensulfiden;
- Extrakten von einem oder mehreren nicht photosynthetisierenden und nicht fruchtkörperbildenden Fadenbakterien;
- Ellagsäure, Ethern davon, Ellagsäuresalzen und Salzen von Ethern davon sowie Ellagsäuretanninen;
- Schwefel, Cadmiumsulfid, Allantoin, Kohlen- oder Holzteeren und Derivaten davon, Undecylensäure, Fumarsäure und Allylaminen wie Terbinafin;
wobei die Antischuppenmittel vorzugsweise in einer Menge im Bereich von 0,01 bis 10 Gew.-%, vorzugsweise von 0,2 bis 5 Gew.-%, noch besser von 0,5 bis 3 Gew.-%, bezogen auf das Gesamtgewicht der Zusammensetzung, vorliegen.

15. Zusammensetzung nach einem der vorhergehenden Ansprüche, umfassend Wasser in einer Konzentration im Bereich von 20 bis 80 Gew.-%, insbesondere 35 bis 75 Gew.-% oder sogar von 40 bis 70 Gew.-%, besser von 45 bis 65 Gew.-%, noch besser von 50 bis 65 Gew.-%, bezogen auf das Gesamtgewicht der Zusammensetzung.

16. Verfahren zur kosmetischen Behandlung, insbesondere zur Pflege, Reinigung und/oder Konditionierung von Keratinmaterialien, insbesondere der Haare, das darin besteht, dass man eine Zusammensetzung gemäß einem der Ansprüche 1 bis 15 auf die Keratinmaterialien aufbringt und nach einer fakultativen Einwirkungszeit gegebenenfalls spült, beispielsweise mit Wasser.

## Claims

1. Aqueous cosmetic composition which is in a flexible solid form, comprising:
(i) one or more anionic surfactants, in a total amount ranging from 15% to 60% by weight relative to the total weight of the composition,
said composition comprising at least one anionic surfactant chosen from acyl isethionates, preferably having an acyl group comprising from 6 to 30 carbon atoms, better still from 12 to 24, even better still from 16 to 22 carbon atoms;
the total amount of acyl isethionates in the composition ranging from 12% to 35% by weight relative to the total weight of the composition;
(ii) one or more polymeric conditioning agents chosen from non-silicone cationic polymers, non-silicone amphoteric polymers, silicones, in particular amino silicones, and mixtures thereof;
said composition comprising at least 20% by weight of water relative to the total weight of the composition; and
said composition having a threshold stress at 25°C greater than or equal to 100 Pa, and/or having a penetration force at 25°C greater than or equal to 210 g, and/or having a viscoelastic spectrum at 25°C, measured between 10⁻² Hz and 100 Hz, such that there is no crossover point between the curves G' and G"; G' always being strictly greater than G".

2. Composition according to Claim 1, having a threshold stress at 25°C ranging from 100 to 900 Pa.

3. Composition according to either of the preceding claims, having a penetration force at 25°C ranging from 210 to 900 g, better still ranging from 350 to 800 g.

4. Composition according to one of the preceding claims, in which the acyl isethionate surfactants correspond to the formula R-C(O)-O-CH₂CH₂SO₃M, in which R-C(O) is an acyl group preferably comprising from 6 to 30 carbon atoms, better still from 12 to 24, even better still from 16 to 22 carbon atoms, and M denotes a cosmetically acceptable counterion; and preferably are chosen from cocoyl isethionates and/or lauroyl isethionates.

5. Composition according to one of the preceding claims, comprising one or more additional sulfate, sulfonate or carboxylate anionic surfactants, preferably chosen from:
- C₆-C₂₄ and notably C₁₂-C₂₀ alkyl sulfates;
- C₆-C₂₄ and notably C₁₂-C₂₀ alkyl ether sulfates, preferably comprising from 2 to 20 ethylene oxide units;
- C₆-C₂₄ and notably C₁₂-C₂₀ acyl glutamates, notably stearoyl glutamates;
- C₆-C₂₄ and notably C₁₂-C₂₀ acyl sarcosinates, notably palmitoyl sarcosinates;
- C₆-C₂₄ and notably C₁₂-C₂₀ acyl lactylates, notably behenoyl lactylates;
- (C₆-C₂₄) alkyl ether carboxylates, preferably (C₁₂-C₂₀)alkyl ether carboxylates;
- C₆-C₂₄ and notably C₁₂-C₂₀ alkyl sulfosuccinates, notably lauryl sulfosuccinates.

6. Composition according to one of the preceding claims, in which the total amount of anionic surfactant(s) ranges from 20% to 60% by weight, preferentially from 25% to 60% by weight, or even from 30% to 60% by weight, relative to the total weight of the composition.

7. Composition according to one of the preceding claims, in which the total amount of acyl isethionates in the composition ranges from 15% to 35% by weight, even better still from 20% to 35% by weight, relative to the total weight of the composition.

8. Composition according to one of the preceding claims, in which the polymeric conditioning agents are chosen from non-silicone cationic polymers and silicones, notably amino silicones; and even better still mixtures thereof.

9. Composition according to one of the preceding claims, in which the polymeric conditioning agents are chosen from:
(a) homopolymers or copolymers derived from acrylic or methacrylic esters or amides and comprising at least one of the units having the following formula: 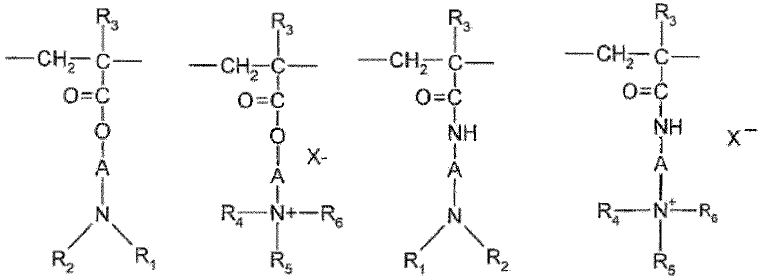 in which:
- R₃, which may be identical or different, denote a hydrogen atom or a CH₃ radical;
- A, which may be identical or different, represent a linear or branched, divalent alkyl group containing from 1 to 6 carbon atoms, preferably 2 or 3 carbon atoms, or a hydroxyalkyl group containing from 1 to 4 carbon atoms;
- R₄, R₅ and R₆, which may be identical or different, represent an alkyl group having from 1 to 18 carbon atoms or a benzyl radical; preferably an alkyl group having from 1 to 6 carbon atoms;
- R₁ and R₂, which may be identical or different, represent a hydrogen atom or an alkyl group having from 1 to 6 carbon atoms, preferably methyl or ethyl;
- X denotes an anion derived from a mineral or organic acid, such as a methosulfate anion or a halide such as chloride or bromide;
(b) cationic polysaccharides, in particular celluloses and galactomannan gums which are cationic;
(c) cyclopolymers of alkyldiallylamine or dialkyldiallylammonium, such as homopolymers or copolymers comprising, as main constituent of the chain, units corresponding to formula (I) or (II): 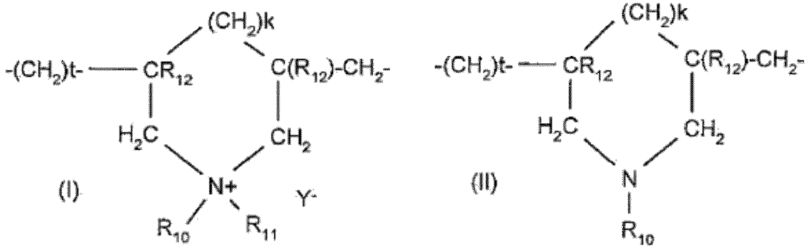 in which
- k and t are equal to 0 or 1, the sum k + t being equal to 1;
- R₁₂ denotes a hydrogen atom or a methyl radical;
- R₁₀ and R₁₁, independently of one another, denote an alkyl group having from 1 to 6 carbon atoms, a hydroxyalkyl group in which the alkyl group contains 1 to 5 carbon atoms, a C₁-C₄ amidoalkyl group; or else R₁₀ and R₁₁ can denote, together with the nitrogen atom to which they are attached, heterocyclic groups, such as piperidinyl or morpholinyl; R₁₀ and R₁₁, independently of one another, preferably denote an alkyl group having from 1 to 4 carbon atoms;
- Y⁻ is an anion such as bromide, chloride, acetate, borate, citrate, tartrate, bisulfate, bisulfite, sulfate or phosphate;
(d) quaternary polymers of vinylpyrrolidone and of vinylimidazole;
(e) polydialkylsiloxanes, in particular polydimethylsiloxanes (PDMSs);
(f) amino silicones, and in particular corresponding to the formula below: 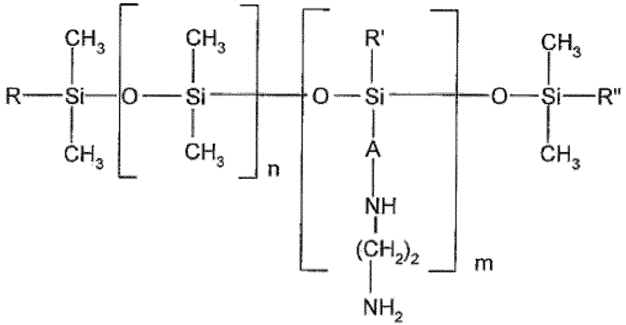 in which R, R' and R", which may be identical or different, denote a C₁-C₄ alkyl radical, preferably CH₃; a C₁-C₄ alkoxy radical, preferably methoxy; or OH; A represents a linear or branched C₁-C₈, preferably C₃-C₆, alkylene radical; m and n are integers dependent on the molecular weight and the sum of which is between 1 and 2000.

10. Composition according to one of the preceding claims, in which the polymeric conditioning agents are present in an amount ranging from 0.001% to 20% by weight, preferentially from 0.1% to 10% by weight, even better still from 0.2% to 8% by weight, or even from 0.4% to 5% by weight, preferentially from 0.5% to 5% by weight, relative to the total weight of the composition.

11. Composition according to one of the preceding claims, also comprising one or more non-polymeric conditioning agents, which can be chosen from cationic surfactants, fatty esters other than triglycerides, fatty alcohols, plant oils, liquid C₆-C₁₆ hydrocarbons, hydrocarbons having more than 16 carbon atoms, ceramides, plant or animal waxes, and mixtures thereof.

12. Composition according to one of the preceding claims, also comprising one or more types of solid particles, which are generally insoluble in water; preferably chosen from:
- micronized fruit kernel particles;
- pyridinethione salts, in particular the calcium, magnesium, barium, strontium, zinc, cadmium, tin and zirconium salts;
- crosslinked or non-crosslinked polymer particles; and in particular polyamide powders; powders of acrylic polymers, in particular of crosslinked poly(sodium acrylate), of poly(methyl methacrylate); powders of acrylic copolymers, in particular of poly(methyl methacrylate/ethylene glycol dimethacrylate), of poly(allyl methacrylate/ethylene glycol dimethacrylate), of copolymer of ethylene glycol dimethacrylate/lauryl methacrylate, of polyacrylate/alkyl acrylate; polystyrene powders; polyethylene powders, in particular polyethylene/acrylic acid powders; silicone resin microbeads;
- metal particles, oxides, inorganic salts, carbides, nitrides, borides, sulfides and hydroxides; and in particular clays, silicates, alumina, silica, kaolin and hydroxapatite;
- organic or mineral coloured pigments, or else non-coloured nacreous agents;
said solid particles preferably being present in an amount ranging from 0.001% to 15% by weight, preferentially from 0.1% to 10% by weight, even better still from 0.5% to 8% by weight, relative to the total weight of the composition.

13. Composition according to one of the preceding claims, also comprising one or more polyols, preferably of formula: 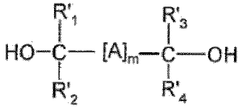 in which:
- R'₁, R'₂, R'₃ and R'₄ denote, independently of one another, a hydrogen atom, a C₁-C₆ alkyl radical or a C₁-C₆ mono- or polyhydroxyalkyl radical;
- A denotes a linear or branched alkylene radical comprising from 1 to 18 carbon atoms, and optionally from 1 to 9 oxygen atoms, but no hydroxyl group;
- m denotes 0 or 1;
preferably, said polyols being present in an amount ranging from 0.1% to 60% by weight, preferentially from 0.5% to 50% by weight, even better still from 1% to 30% by weight, relative to the total weight of the composition.

14. Composition according to one of the preceding claims, also comprising one or more antidandruff agents; in particular chosen from:
- pyridinethione salts;
- 1-hydroxy-2-pyrrolidone derivatives;
- 2,2'-dithiobis(pyridine-N-oxide);
- trihalocarbamides;
- triclosan;
- azole compounds;
- antifungal polymers;
- selenium sulfides;
- extracts of one or more non-photosynthetic, non-fruiting filamentous bacteria;
- ellagic acid, ethers thereof, ellagic acid salts and the salts of ethers thereof; and also ellagic acid tannins;
- sulfur, cadmium sulfide, allantoin, coal or wood tars and derivatives thereof, undecylenic acid, fumaric acid, and allylamines such as terbinafine;
said antidandruff agents preferably being present in an amount ranging from 0.01% to 10% by weight, preferentially from 0.2% to 5% by weight, even better still from 0.5% to 3% by weight, relative to the total weight of the composition.

15. Composition according to one of the preceding claims, comprising water at a concentration ranging from 20% to 80% by weight, in particular from 35% to 75% by weight, or even from 40% to 70% by weight, better still from 45% to 65% by weight and even better still from 50% to 65% by weight, relative to the total weight of the composition.

16. Cosmetic treatment process, in particular for caring for, cleansing and/or conditioning keratin materials, in particular the hair, which consists in applying a composition as defined in one of Claims 1 to 15 to said keratin materials, and in optionally performing a rinsing operation, for example with water, after an optional leave-on time.
